# EUROPEAN PATENT APPLICATION

(11) **EP 3 569 593 A1**
(43) Date of publication of application: **20.11.2019**
(21) Application number: 18173329.6
(22) Date of filing: 18.05.2018
(51) Int. Cl.: C07D 213/75, C07C 269/04, C07C 209/60, C07C 213/08, C07C 227/10, C07C 253/30, C07F 7/08, C07F 9/40

(54) **PRODUCTION OF AMINES VIA A HYDROAMINOMETHYLATION REACTION**

(71) Applicant: Universität Wien, 1010 Vienna (AT)
(72) Inventor: MAULIDE, Nuno, 1090 Vienna (AT); SHAABAN, Saad, 1090 Vienna (AT); GONCALVES, Carlos, 1090 Vienna (AT); TONA, Veronica, 1090 Vienna (AT); KAISER, Daniel, 1090 Vienna (AT)
(74) Representative: Vossius & Partner Patentanwälte Rechtsanwälte mbB

(57) **Abstract**

Provided is a process for producing an amine via a hydroaminomethylation reaction of a non-aromatic C-C double bond or C-C triple bond, said process comprising
a step of reacting a compound comprising a non-aromatic C-C double bond or C-C triple bond with a reactive component which is obtainable by combining an aminal with an acidic medium comprising trifluoroacetic acid,
wherein the aminal contains two amino groups independently selected from a secondary and a tertiary amino group that are linked by a methylene group, and at least one of the amino groups carries a hydrogen atom at a carbon atom bound in α-position to its nitrogen atom.

## Description

The present invention relates to a process for producing an amine via a hydroaminomethylation reaction of a non-aromatic C-C double bond or triple bond.

Amines are key structural elements in our life, as they constitute core elements of a variety of biologically active agents and crucial high-performance materials. Accordingly, the synthesis of amino compounds from abundant and readily available substrates is of crucial importance to chemistry, biology, medicine and materials science. In particular, the synthesis of amines starting from substituted or non-substituted alkenes and alkynes is of relevance. A direct functionalization of such alkenes and alkynes can be achieved by a hydroaminomethylation reaction, addition of hydrogen and a methylamine-unit across an olefin.

State-of-the-art protocols for hydroaminomethylation of alkenes rely largely on transition-metal catalysis, which enables hydroaminomethylation under highly designed and controlled conditions. Several metal-catalyzed hydroaminomethylations of alkenes with amines have been reported in recent years. The intermolecular addition of the α-C-H bonds of a dialkylamine to an unactivated olefin in the presence of a chloro amido tantalum complex is described in "Hydroaminoalkylation of unactivated olefins with dialkylamines" of Herzon, S. B. & Hartwig, J. F., J. Am. Chem. Soc. 2008, 130, 14940-14941. A catalytic hydroaminomethylation of an alkene with dimethylamine using a titanium catalyst is described in "Dimethylamine as a substrate in hydroaminoalkylation reactions" of Bielefeld, J. & Doye, S., Angew. Chem. Int. Ed. 2017, 56, 15155-15158. While proceeding under catalytic conditions and employing simple amines as starting materials, metal-catalyzed hydroaminomethylation reactions, dominated by group 4 and group 5 metal catalysts, have shown some restrictions in terms of selectivity and functional group tolerance.

An alternative approach to the hydroaminomethylation of alkenes lies in photocatalysis or dual catalysis using transition-metal and photocatalysis. α-Aminoalkyl radicals have been added to electron-deficient alkenes by visible-light-mediated electron transfer using transition metal polypyridyl complexes as photocatalysts (Miyake, Y., Nakajima, K. & Nishibayashi, Y., "Visible-light-mediated utilization of α-aminoalkyl radicals: addition to electron-deficient alkenes using photoredox catalysts", J. Am. Chem. Soc. 2012, 134, 3338-3341). The coupling of photoredox-generated α-amino radical species with conjugated dienes using a unified cobalt and iridium catalytic system in order to access a variety of useful homoallylic amines from simple commercially available starting materials is described in "A mild hydroaminoalkylation of conjugated dienes using a unified cobalt and photoredox catalytic system" of Thullen, S. M. & Rovis, T., J. Am. Chem. Soc. 2017, 139, 15504-15508. Even though mild reaction conditions can be applied, photocatalytic hydroaminomethylation reactions are limited to specific substrates.

In order to address these challenges, the present inventors became interested in the development of a redox-neutral hydroaminomethylation reaction circumventing the need for early transition-metal catalysis and photocatalysis. In a hydroaminomethylation reaction of 2-phenyl-2-norbornene with formaldehyde and dimethylamine in acetic acid, a mixture of compounds best explained by a 1,5-hydride shift in one intermediate step is produced (Manninen, K. & Haapala, J., "The Reaction of 2-Phenyl-2-norbornene with Formaldehyde and Dimethylamine. Additional Evidence for the Occurrence of a 1,5-Hydride Shift during the Aminomethylation of a Strained Bicycloalkene Structure", Acta Chem. Scand. B, 1974, 28, 433-440). A hydroaminomethylation of 1-alkenes with an iminium ion generated by the action of phosphoric or sulfuric acid on (tetramethyldiamino)methane in acetic acid results in secondary amines and unsaturated tertiary amines (Cohen, T. & Onopchenko, A., "Competing Hydride Transfer and Ene Reactions in the Aminoalkylation of 1-Alkenes with N,N-Dimethyleniminium Ions. A Literature Correction", J. Org. Chem., 1983, 48, 4531-4537). However, also known redox-neutral hydroaminomethylation reactions suffer from certain limiations, such as formation of elimination products and other side products, low yields and limited functional group tolerance.

In the context of the present invention, it has been found that the use of an aminal as starting material and a trifluoroacetic acid in a hydroaminomethylation reaction of non-aromatic C-C double bond or C-C triple bond can markedly influence this reaction in that competing elimination reactions are suppressed and high product yields, a broad functional group tolerance and linear selectivities are achieved. A broadly applicable, redox-neutral approach to hydroaminomethylation of compounds comprising a non-aromatic C-C double bond or C-C triple bond, wherein cheap, readily available and bench-stable reactants is thus made available. The reported hydroaminomethylation affords the desired amines with excellent functional group tolerance and high regio- and stereoselectivity.

Thus, the present invention provides a process for producing an amine via a hydroaminomethylation reaction of a non-aromatic C-C double bond or a triple bond, said process comprising a step of reacting a compound comprising a non-aromatic C-C double bond or C-C triple bond with a reactive component which is obtainable by combining an aminal with an acidic medium comprising trifluoroacetic acid (TFA), wherein the aminal contains two amino groups independently selected from a secondary and a tertiary amino group that are linked by a methylene group, and at least one of the amino groups carries a hydrogen atom at a carbon atom bound in α-position to its nitrogen atom.

An exemplary hydroaminomethylation reaction of a non-aromatic C-C double bond or C-C triple bond in accordance with the present invention is illustrated by the following reaction scheme 1.

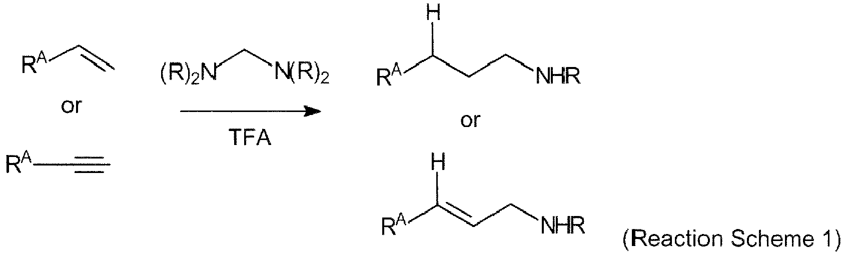

In this reaction, a non-aromatic C-C double bond or C-C triple bond is converted with an aminal in an acidic medium to an amine. The groups R and R^{A} represent any desired atoms or groups, and it should be understood that the compound comprising a non-aromatic C-C double bond or C-C triple bond and the aminal used in the process of the present invention are not limited to the exemplified structures as shown in this scheme.

As noted above, the process of the present invention encompasses a hydroaminomethylation reaction of a non-aromatic C-C double bond or C-C triple bond. Thus, it comprises a step of reacting a compound comprising a non-aromatic C-C double bond or triple bond with a reactive component which is obtainable by combining an aminal with an acidic medium comprising trifluoroacetic acid.

### Aminal

In the process in accordance with the present invention, a compound comprising a non-aromatic C-C double bond or C-C triple bond is reacted with a reactive component which is obtainable by combining an aminal with an acidic medium comprising trifluoroacetic acid.

An aminal, also referred to as a germinal diamine in the art, contains two amino groups bonded to the same carbon atom. In the process in accordance with the invention, the reactive component is obtainable using an aminal which contains two amino groups independently selected from a secondary and a tertiary amino group that are linked via a methylene group, and at least one of the amino groups carries a hydrogen atom at a carbon atom bound in α-position to the nitrogen atom. Preferably, the two amino groups linked via the methylene group are both tertiary amino groups.

As will be understood by the skilled reader, a secondary amino group represents an amino group, wherein two of the three substituents bound to the nitrogen atom are organic groups other than hydrogen. A tertiary amino group represents an amino group wherein the three substituents bound to the nitrogen atom are organic groups other than hydrogen. In the case of the amino groups contained in the aminal used in the process in accordance with the present invention, one of the three substituent positions is kept by the methylene group as an organic group which links the two amino groups. In other words, in the case of a secondary amino group, one further organic group which is not hydrogen is bound to the nitrogen atom besides the methylene group, and in the case of a tertiary amino group, two further organic groups which are not hydrogen are bound to the nitrogen atom besides the methylene group.

Moreover, as noted above, at least one of the amino groups in the aminal carries a hydrogen atom at a carbon atom bound in α-position to the nitrogen atom. As will be understood by the skilled reader, a carbon atom bound in α-position to the nitrogen atom is a carbon atom (generally an sp³-hybridized carbon atom) which is directly bound to the nitrogen atom. The carbon atom bound in α-position to the nitrogen atom is one which is present in addition to the carbon atom of the methylene group linking the amino groups. Thus, the aminal contains a structural unit which may be represented as 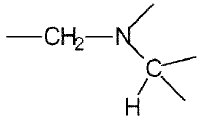 wherein the -CH₂- group represents the methylene group linking the two geminal amino groups of the aminal.

It is noted that the organic groups which are bound to the nitrogen atoms of the amino groups may include known protective groups for amino groups which may be removed after the hydroaminomethylation reaction, such as a benzyl group, an allyl group or a Boc group (i.e. a *tert*-butyloxycarbonyl group).

Preferably, the aminal used to provide the reactive component in accordance with the present invention is represented by the following formula (I): 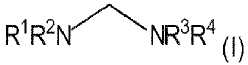 wherein
R¹ and R³ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted;
R² and R⁴ are independently selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted;
and R¹ and R² or R³ and R⁴, respectively, may be bonded to each other to form a ring together with the nitrogen atom to which they are bound, or one of R¹ and R² and one of R³ and R⁴ may be bonded to each other to form a ring together with the methylene group and the nitrogen atoms to which they are bound.

In formula (I), at least one of the amino groups -NR¹R² and -NR³R⁴ carries a hydrogen atom at a carbon atom in α-position to the nitrogen atom. As will be understood by the skilled reader, this carbon atom in α-position to the nitrogen atom, generally an sp³-hybridized carbon atom, may be contained in any of R¹, R², R³ and R⁴. The amino groups -NR¹R² and -NR³R⁴ are preferably both tertiary amino groups, i.e. groups wherein R¹, R², R³ and R⁴ are groups other than hydrogen.

Preferably, R¹, R², R³ and R⁴ are selected such that -NR¹R² and -NR³R⁴ have the same structure. More preferably, R¹, R², R³ and R⁴ are selected such that -NR¹R² and -NR³R⁴ are both tertiary amino groups and that R¹, R², R³ and R⁴ have the same structure.

It is noted that, in the formulae described herein, any hydrogen atoms can generally be replaced by deuterium. This applies not only for formula (I) above, but also to formulae (II) and (III) below.

The aliphatic hydrocarbon group has preferably 1 to 30 carbon atoms, more preferably 1 to 24 carbon atoms, and even more preferably 1 to 12 carbon atoms. The aromatic hydrocarbon group has preferably 6 to 30 carbon atoms, more preferably 6 to 22 carbon atoms, and even more preferably 6 to 14 carbon atoms. The aliphatic heterocyclic group has preferably 2 to 30 carbon atoms, more preferably 2 to 24 carbon atoms, and even more preferably 2 to 12 carbon atoms. The aromatic heterocyclic group has preferably 2 to 30 carbon atoms, more preferably 3 to 22 carbon atoms, and even more preferably 3 to 12 carbon atoms. The aralkyl group has preferably 7 to 30 carbon atoms, more preferably 7 to 24 carbon atoms, and even more preferably 7 to 14 carbon atoms.

The aliphatic heterocyclic group and the aromatic heterocyclic group have independently preferably 1 to 5 heteroatoms, more preferably 1 to 3 heteroatoms. The heteroatom is preferably selected from the group consisting of a nitrogen atom, an oxygen atom and a sulphur atom.

The aliphatic hydrocarbon group, the aromatic hydrocarbon group, the aliphatic heterocyclic group, the aromatic heterocyclic group, alone or in combination, may be substituted with one or more, such as one, two or three, substituents. Exemplary substituents are an aliphatic hydrocarbon group having 1 to 12 carbon atoms, an aromatic hydrocarbon group having 6 to 14 carbon atoms, an aliphatic heterocyclic group having 2 to 12 carbon atoms and 1 to 3 heteroatoms, an aromatic heterocyclic group having 3 to 12 carbon atoms and 1 to 3 heteroatoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn carry one or more, such as one, two or three, substituents selected from an aliphatic hydrocarbon group having 1 to 12 carbon atoms, an aromatic hydrocarbon group having 6 to 14 carbon atoms, an aliphatic heterocyclic group having 2 to 12 carbon atoms and 1 to 3 heteroatoms, an aromatic heterocyclic group having 3 to 12 carbon atoms and 1 to 3 heteroatoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

Suitable examples of an aliphatic hydrocarbon group are a linear or branched alkyl group, a cycloalkyl group, a linear or branched alkenyl group, a cycloalkenyl group and a linear or branched alkynyl group. Suitable examples of an aliphatic heterocyclic group are a heterocycloalkyl group and a heterocycloalkenyl group. The aromatic heterocyclic group may also be referred to as a heteroaromatic group.

A linear alkyl group as referred to herein has preferably 1 to 30 carbon atoms, more preferably 1 to 24 carbon atoms, and even more preferably 1 to 12 carbon atoms. A branched alkyl group as referred to herein has preferably 3 to 30 carbon atoms, more preferably 3 to 24 carbon atoms, and even more preferably 3 to 12 carbon atoms. A cycloalkyl group as referred to herein has preferably 3 to 30 carbon atoms, more preferably 3 to 24 carbon atoms, and even more preferably 3 to 12 carbon atoms. A linear alkenyl group as referred to herein has preferably 2 to 30 carbon atoms, more preferably 2 to 24 carbon atoms, and even more preferably 2 to 12 carbon atoms. A branched alkenyl group as referred to herein has preferably 4 to 30 carbon atoms, more preferably 4 to 24 carbon atoms, and even more preferably 4 to 12 carbon atoms. A cycloalkenyl group as referred to herein has preferably 3 to 30 carbon atoms, more preferably 3 to 24 carbon atoms, and even more preferably 3 to 12 carbon atoms. A linear alkynyl group as referred to herein has preferably 2 to 30 carbon atoms, more preferably 2 to 24 carbon atoms, and even more preferably 2 to 12 carbon atoms. A branched alkynyl group as referred to herein has preferably 4 to 30 carbon atoms, more preferably 4 to 24 carbon atoms, and even more preferably 4 to 12 carbon atoms. An aromatic hydrocarbon group as referred to herein has preferably 6 to 30 carbon atoms, more preferably 6 to 22 carbon atoms, and even more preferably 6 to 14 carbon atoms. A heterocycloalkyl group as referred to herein has preferably 2 to 30 carbon atoms and 1 to 5 heteroatoms, more preferably 2 to 24 carbon atoms and 1 to 5 heteroatoms, and even more preferably 2 to 12 carbon atoms and 1 to 3 heteroatoms. A heterocycloalkenyl group as referred to herein has preferably 2 to 30 carbon atoms and 1 to 5 heteroatoms, more preferably 2 to 24 carbon atoms and 1 to 5 heteroatoms, and even more preferably 2 to 12 carbon atoms and 1 to 3 heteroatoms. A heteroaromatic hydrocarbon group as referred to herein has preferably 2 to 30 carbon atoms and 1 to 5 heteroatoms, more preferably 3 to 22 carbon atoms and 1 to 5 heteroatoms, and even more preferably 3 to 12 carbon atoms and 1 to 3 heteroatoms. The heteroatom is preferably selected from the group consisting of a nitrogen atom, an oxygen atom and a sulphur atom.

In formula (I), R¹ and R³ are preferably independently selected from the group consisting of hydrogen, a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted;
R² and R⁴ are preferably independently selected from the group consisting of a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted;
and R¹ and R² or R³ and R⁴, respectively, may be bonded to each other to form a ring together with the nitrogen atom to which they are bound.

Also with these preferred groups R¹, R², R³ and R⁴, at least one of the amino groups -NR¹R² and -NR³R⁴ carries a hydrogen atom at a carbon atom in α-position to the nitrogen atom. As will be understood by the skilled reader, this carbon atom in α-position to the nitrogen atom, generally an sp³-hybridized carbon atom, may be contained in any of R¹, R², R³ and R⁴. The amino groups -NR¹R² and -NR³R⁴ are preferably both tertiary amino groups. Preferably, R¹, R², R³ and R⁴ are selected such that -NR¹R² and -NR³R⁴ have the same structure. More preferably, R¹, R², R³ and R⁴ are selected such that -NR¹R² and -NR³R⁴ are both tertiary amino groups and that R¹, R², R³ and R⁴ have the same structure.

The optionally substituted groups may be substituted by one or more, such as one, two or three, substituents. Exemplary substituents for the optionally substituted groups are selected from an alkyl group having 1 to 12 carbon atoms, a cycloalkyl group having 3 to 12 carbon atoms, a heterocycloalkyl group having 2 to 12 carbon atoms, an alkenyl group having 2 to 12 carbon atoms, a cycloalkenyl group having 3 to 12 carbon atoms, an alkynyl group having 2 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may carry in turn one or more, such as one, two or three, substituents selected from an alkyl group having 1 to 12 carbon atoms, a cycloalkyl group having 3 to 12 carbon atoms, a heterocycloalkyl group having 2 to 12 carbon atoms, an alkenyl group having 2 to 12 carbon atoms, a cycloalkenyl group having 3 to 12 carbon atoms, an alkynyl group having 2 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

In formula (I), more preferably R¹, R², R³ and R⁴ are preferably independently selected from the group consisting of a linear or branched alkyl group which may be substituted, a cycloalkyl group which may be substituted, a heterocycloalkyl group which may be substituted, a linear or branched alkenyl group which may be substituted, a cycloalkenyl group which may be substituted, an aromatic hydrocarbon group which may be substituted, an aralkyl group which may be substituted and a heteroaromatic hydrocarbon group which may be substituted, and wherein R¹ and R² or R³ and R⁴, respectively, may be bonded to each other to form a ring together with the nitrogen atom to which they are bound.

Also with these more preferred groups R¹, R², R³ and R⁴, at least one of the amino groups -NR¹R² and -NR³R⁴ carries a hydrogen atom at a carbon atom in α-position to the nitrogen atom. As will be understood by the skilled reader, this carbon atom in α-position to the nitrogen atom, generally an sp³-hybridized carbon atom, may be contained in any of R¹, R², R³ and R⁴. Preferably, R¹, R², R³ and R⁴ are selected such that -NR¹R² and -NR³R⁴ have the same structure. More preferably, R¹, R², R³ and R⁴ have the same structure.

The optionally substituted groups may be substituted by one or more, such as one, two or three, substituents. Exemplary substituents for the optionally substituted groups are selected from the group consisting of an alkyl group having 1 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

In formula (I), R¹, R², R³ and R⁴ are even more preferably independently selected from the group consisting of methyl, ethyl, propyl, isopropyl, butyl, isobutyl, benzyl and allyl, and R¹ and R² or R³ and R⁴, respectively, may be bonded to each other to form a ring together with the nitrogen atom to which they are bound. As a preferred ring, a piperidin-1-yl ring may be mentioned.

Also these even more preferred groups R¹, R², R³ and R⁴ are preferably selected such that -NR¹R² and -NR³R⁴ have the same structure. More preferably, R¹, R², R³ and R⁴ have the same structure.

The aminal used in the context of the present invention can be prepared by conventional methods of synthesis, e.g. by reacting an amine, optionally a protected amine, with formaldehyde.

### Compound comprising a non-aromatic C-C double bond

As noted above, the invention encompasses two aspects, i.e. a process encompassing a hydroaminomethylation reaction of a non-aromatic C-C double bond as a first aspect and a process encompassing a hydroaminomethylation reaction of a C-C triple bond as a second aspect.

The compound comprising a non-aromatic C-C double bond (i.e. a non-aromatic double bond which links two carbon atoms) which is subjected to the reaction with the reactive component in accordance with the first aspect of the present invention is an organic compound which may comprise one non-aromatic C-C double bond or multiple, i.e. two or more, non-aromatic C-C double bonds. If the compound comprises multiple C-C double bonds, the double bonds may be conjugated double bonds. In a case of multiple C-C double bonds, the process in accordance with the invention may be applicable for multiple hydroaminomethylation reactions in a single compound.

Preferably, the compound comprising a non-aromatic C-C double bond comprises one or two non-aromatic C-C double bonds, more preferably one non-aromatic C-C double bond.

Since the non-aromatic C-C double bond represents the relevant functional feature of the compound subjected to the hydroaminomethylation reaction, the compound comprising the non-aromatic C-C double bond may also be referred to as an "alkene" herein. Unless indicated otherwise, this reference to an alkene includes the optional presence of optional subsitituents bound to the hydrocarbon core structure of the alkene.

The structure of the compound comprising a non-aromatic C-C double bond is not particularly limited. For example, the non-aromatic C-C double bond may form part of a linear, a branched or a ring structure, or of a linear, a branched or a ring substructure in the compound comprising the non-aromatic C-C double bond. In addition to the non-aromatic C-C double bond, the compound may comprise one or more additional functional groups and/or aromatic double bonds. Moreover, the reference herein to a reaction of a non-aromatic C-C double bond or C-C triple bond does not use the "or" in an exclusive manner, so that the two types of bonds may occur in a single compound.

Preferably, the non-aromatic C-C double bond (or the non-aromatic C-C double bonds, if multiple double bonds are present) is/are selected from a non-substituted, a monosubstituted, a disubstituted and a trisubstituted non-aromatic C-C double bond. More preferably, the non-aromatic C-C double bond (or the non-aromatic C-C double bonds, if multiple double bonds are present) is/are selected from a monosubstited and a disubstituted non-aromatic C-C double bond. As will be understood by the skilled reader, a non-substituted C-C double bond is a C-C double bond wherein neither one of the two carbon atoms linked by the double bond carries a substituent other than a hydrogen (i.e. the compound comprising the non-substituted C-C double bond is ethene). In a monsubstituted C-C double bond, one of the carbon atoms linked by the double bond carries one substituent other than hydrogen. Reference to a disubstituted C-C double bond herein refers, unless specifically indicated otherwise, to a C-C double bond wherein each of the two carbon atoms linked by the double bond carries one substituent other than a hydrogen atom. Accordingly, in a trisubstituted C-C double bond, one of the carbon atoms linked by the double bond carries two substituents other than hydrogen, the other one carries one substituent other than hydrogen. Preferably, the substituents other than hydrogen are substituted or non-substituted hydrocarbon groups.

A preferred, exemplary compound comprising a non-aromatic C-C double bond for use in the process in accordance with the present invention is represented by the following formula (II): 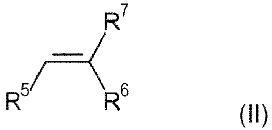 wherein R⁵, R⁶ and R⁷ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted, and of combinations thereof, such as an aralkyl group which may be substituted, and wherein R⁵ and R⁶ may be bonded to each other to form a ring together with the carbon atoms to which they are bound.

The aliphatic hydrocarbon group has preferably 1 to 30 carbon atoms, more preferably 1 to 24 carbon atoms, and even more preferably 1 to 12 carbon atoms. The aromatic hydrocarbon group has preferably 6 to 30 carbon atoms, more preferably 6 to 22 carbon atoms, and even more preferably 6 to 14 carbon atoms. The aliphatic heterocyclic group has preferably 2 to 30 carbon atoms, more preferably 2 to 24 carbon atoms, and even more preferably 2 to 12 carbon atoms. The aromatic heterocyclic group has preferably 2 to 30 carbon atoms, more preferably 3 to 22 carbon atoms, and even more preferably 3 to 12 carbon atoms. The aralkyl group has preferably 7 to 30 carbon atoms, more preferably 7 to 24 carbon atoms, and even more preferably 7 to 14 carbon atoms.

The aliphatic heterocyclic group and the aromatic heterocyclic group contain independently preferably 1 to 5 heteroatoms, more preferably 1 to 3 heteroatoms. The heteroatom is preferably selected from a nitrogen atom, an oxygen atom and a sulphur atom.

The aliphatic hydrocarbon group, the aromatic hydrocarbon group, the aliphatic heterocyclic group, the aromatic heterocyclic group, alone or in combination, may be substituted by one or more, such as one two or three, substituents. Exemplary substituents are aliphatic hydrocarbon groups having 1 to 12 carbon atoms, aromatic hydrocarbon groups having 6 to 14 carbon atoms, aliphatic heterocyclic groups having 2 to 12 carbon atoms and 1 to 3 heteroatoms, aromatic heterocyclic groups having 3 to 12 carbon atoms and 1 to 3 heteroatoms, halogen atoms, oxo groups, hydroxyl groups, cyano groups, amino groups, carbamoyl groups, carboxyl groups, alkoxy groups having 1 to 10 carbon atoms, alkoxycarbonyl groups having 2 to 10 carbon atoms, alkanoyloxy groups having 2 to 10 carbon atoms, phosphonate groups and trialkylsilyl groups having 3 to 30 carbon atoms. Where suitable, these exemplary substituents may in turn be substituted by one or more, such as one, two or three, substituents selected from aliphatic hydrocarbon groups having 1 to 12 carbon atoms, aromatic hydrocarbon groups having 5 to 14 carbon atoms, aliphatic heterocyclic groups having 2 to 12 carbon atoms and 1 to 3 heteroatoms, aromatic heterocyclic groups having 3 to 12 carbon atoms and 1 to 3 heteroatoms, halogen atoms, oxo groups, hydroxyl groups, cyano groups, amino groups, carbamoyl groups, carboxyl groups, alkoxy groups having 1 to 10 carbon atoms, alkoxycarbonyl groups having 2 to 10 carbon atoms, alkanoyloxy groups having 2 to 10 carbon atoms, phosphonate groups and trialkylsilyl groups having 3 to 30 carbon atoms.

Suitable examples of an aliphatic hydrocarbon group are a linear or branched alkyl group, a cycloalkyl group, a linear or branched alkenyl group, a cycloalkenyl group and a linear or branched alkynyl group. Suitable examples of an aliphatic heterocyclic group are a heterocycloalkyl group and a heterocycloalkenyl group. The aromatic heterocyclic group may also be referred to as a heteroaromatic group.

A linear alkyl group as referred to herein has preferably 1 to 30 carbon atoms, more preferably 1 to 24 carbon atoms, and even more preferably 1 to 12 carbon atoms. A branched alkyl group as referred to herein has preferably 3 to 30 carbon atoms, more preferably 3 to 24 carbon atoms, and even more preferably 3 to 12 carbon atoms. A cycloalkyl group as referred to herein has preferably 3 to 30 carbon atoms, more preferably 3 to 24 carbon atoms, and even more preferably 3 to 12 carbon atoms. A linear alkenyl group as referred to herein has preferably 2 to 30 carbon atoms, more preferably 2 to 24 carbon atoms, and even more preferably 2 to 12 carbon atoms. A branched alkenyl group as referred to herein has preferably 4 to 30 carbon atoms, more preferably 4 to 24 carbon atoms, and even more preferably 4 to 12 carbon atoms. A cycloalkenyl group as referred to herein has preferably 3 to 30 carbon atoms, more preferably 3 to 24 carbon atoms, and even more preferably 3 to 12 carbon atoms. A linear alkynyl group as referred to herein has preferably 2 to 30 carbon atoms, more preferably 2 to 24 carbon atoms, and even more preferably 2 to 12 carbon atoms. A branched alkynyl group as referred to herein has preferably 4 to 30 carbon atoms, more preferably 4 to 24 carbon atoms, and even more preferably 4 to 12 carbon atoms. An aromatic hydrocarbon group as referred to herein has preferably 6 to 30 carbon atoms, more preferably 6 to 22 carbon atoms, and even more preferably 6 to 14 carbon atoms. A heterocycloalkyl group as referred to herein has preferably 2 to 30 carbon atoms and 1 to 5 heteroatoms, more preferably 2 to 24 carbon atoms and 1 to 5 heteroatoms, and even more preferably 2 to 12 carbon atoms and 1 to 3 heteroatoms. A heterocycloalkenyl group as referred to herein has preferably 2 to 30 carbon atoms and 1 to 5 heteroatoms, more preferably 2 to 24 carbon atoms and 1 to 5 heteroatoms, and even more preferably 2 to 12 carbon atoms and 1 to 3 heteroatoms. A heteroaromatic hydrocarbon group as referred to herein has preferably 2 to 30 carbon atoms and 1 to 5 heteroatoms, more preferably 3 to 22 carbon atoms and 1 to 5 heteroatoms, and even more preferably 3 to 12 carbon atoms and 1 to 3 heteroatoms. The heteroatom is preferably selected from the group consisting of a nitrogen atom, an oxygen atom and a sulphur atom.

In formula (II), R⁵, R⁶ and R⁷ are preferably independently selected from the group consisting of hydrogen, a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted, and R⁵ and R⁶ may be bonded to each other to form a ring together with the carbon atoms to which they are bound.

The optionally substituted groups may be substituted by one or more, such as one, two or three, substituents. Exemplary substituents for the optionally substituted groups are selected from an alkyl group having 1 to 12 carbon atoms, a cycloalkyl group having 3 to 12 carbon atoms, a heterocycloalkyl group having 2 to 12 carbon atoms, an alkenyl group having 2 to 12 carbon atoms, a cycloalkenyl group having 3 to 12 carbon atoms, an alkynyl group having 2 to 12 carbon atoms, an aromatic hydrocarbon group having 6 to 14 carbon atoms, a heteroaromatic group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn carry one or more, such as one, two or three, substituents selected from an alkyl group having 1 to 12 carbon atoms, a cycloalkyl group having 3 to 12 carbon atoms, a heterocycloalkyl group having 2 to 12 carbon atoms, an alkenyl group having 2 to 12 carbon atoms, a cycloalkenyl group having 3 to 12 carbon atoms, an alkynyl group having 2 to 12 carbon atoms, an aromatic hydrocarbon group having 6 to 14 carbon atoms, a heteroaromatic group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

In formula (II), more preferably R⁵ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic hydrocarbon group which may be substituted; and either
(i) R⁶ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic group which may be substituted; R⁵ and R⁶ may be bonded to each other to form a ring together with the carbon atoms to which they are bound; and R⁷ is selected from the group consisting of hydrogen and methyl, and is more preferably hydrogen; or
(ii) R⁷ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic group which may be substituted; and R⁶ is selected from the group consisting of hydrogen and methyl, and is more preferably hydrogen.

Also in this case, the optionally substituted groups may be substituted by one or more, such as one, two or three, substituents. Exemplary substituents for the optionally substituted groups are selected from an alkyl group having 1 to 12 carbon atoms, a cycloalkyl group having 3 to 12 carbon atoms, a heterocycloalkyl group having 2 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn carry one or more, such as one, two or three, substituents selected from an alkyl group having 1 to 12 carbon atoms, a cycloalkyl group having 3 to 12 carbon atoms, a heterocycloalkyl group having 2 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

In formula (II), even more preferably R⁵ is selected from the group consisting of hydrogen, an alkyl group which may be substituted, a cycloalkyl group which may be substituted; and either
(i) R⁶ is selected from the group consisting of hydrogen, an alkyl group which may be substituted, a cycloalkyl group which may be substituted, an aromatic hydrocarbon group which may be substituted and an aralkyl group which may be substituted; and R⁵ and R⁶ may be bonded to each other to form a ring together with the carbon atoms to which they are bound; and R⁷ is selected from the group consisting of hydrogen and methyl, and is still more preferably hydrogen; or
(ii) R⁷ is selected from the group consisting of hydrogen, an alkyl group which may be substituted, a cycloalkyl group which may be substituted, an aromatic hydrocarbon group which may be substituted and an aralkyl group which may be substituted; and R⁶ is selected from the group consisting of hydrogen and methyl, and is still more preferably hydrogen.

The optionally substituted groups may be substituted by one or more, such as one, two or three, substituents. Exemplary substituents for the optionally substituted groups are selected from an alkyl group having 1 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn carry one or more, such as one, two or three, substituents selected from an alkyl group having 1 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

### Compound comprising a C-C triple bond

The compound comprising a C-C triple bond (i.e. a triple bond which links two carbon atoms) which is subjected to the reaction with the reactive component in accordance with a second aspect of the present invention is an organic compound which may comprise one C-C triple bond or multiple, i.e. two or more, C-C triple bonds. As will be understood by the skilled reader, the triple bond is also a non-aromatic bond. In a case of multiple C-C triple bonds, the process in accordance with the invention may be applicable for multiple hydroaminomethylation reactions in a single compound.

Preferably, the compound comprising a C-C triple bond comprises one or two C-C triple bonds, more preferably one C-C triple bond.

Since the C-C triple bond represents the relevant functional feature of the compound subjected to the hydroaminomethylation reaction in accordance with the second aspect of the invention, the compound comprising the C-C triple bond may also be referred to as an "alkyne" herein. Unless indicated otherwise, this reference to an alkyne includes the presence of optional subsitituents bound to the hydrocarbon core structure of the alkyne.

The structure of the compound comprising a C-C triple bond is not particularly limited. For example, the C-C triple bond may form part of a linear, a branched or a ring structure, or of a linear, a branched or a ring substructure in the compound comprising the C-C triple bond. In addition to the C-C triple bond, the compound may comprise one or more further functional groups and/or aromatic double bonds. Moreover, the reference herein to a reaction of a non-aromatic C-C double bond or C-C triple bond does not use the "or" in an exclusive manner, so that the two types of bonds may occur in a single compound.

A preferred, exemplary compound comprising a C-C triple bond for use in the process in accordance with the present invention is represented by the following formula (III): 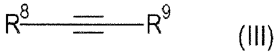 wherein R⁸ and R⁹ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted, and wherein R⁸ and R⁹ may be bonded to each other to form a ring together with the carbon atoms to which they are bound.

The aliphatic hydrocarbon group has preferably 1 to 30 carbon atoms, more preferably 1 to 24 carbon atoms, and even more preferably 1 to 12 carbon atoms. The aromatic hydrocarbon group has preferably 6 to 30 carbon atoms, more preferably 6 to 22 carbon atoms, and even more preferably 6 to 14 carbon atoms. The aliphatic heterocyclic group has preferably 2 to 30 carbon atoms, more preferably 2 to 24 carbon atoms, and even more preferably 2 to 12 carbon atoms. The aromatic heterocyclic group has preferably 2 to 30 carbon atoms, more preferably 3 to 22 carbon atoms, and even more preferably 3 to 12 carbon atoms. The aralkyl group has preferably 7 to 30 carbon atoms, more preferably 7 to 24 carbon atoms, and even more preferably 7 to 14 carbon atoms.

The aliphatic heterocyclic group and the aromatic heterocyclic group contain independently preferably 1 to 5 heteroatoms, more preferably 1 to 3 heteroatoms. The heteroatom may be selected from the group consisting of a nitrogen atom, an oxygen atom and a sulphur atom.

The aliphatic hydrocarbon group, the aromatic hydrocarbon group, the aliphatic heterocyclic group, the aromatic heterocyclic group, alone or in combination, may be substituted by one or more, such as one, two or three, substituents. Exemplary substituents are an aliphatic hydrocarbon group having 1 to 12 carbon atoms, an aromatic hydrocarbon group having 5 to 14 carbon atoms, an aliphatic heterocyclic group having 2 to 12 carbon atoms and 1 to 3 heteroatoms, an aromatic heterocyclic group having 3 to 12 carbon atoms and 1 to 3 heteroatoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn be substituted by one or more, such as one, two or three, substituents selected from an aliphatic hydrocarbon group having 1 to 12 carbon atoms, an aromatic hydrocarbon group having 5 to 14 carbon atoms, an aliphatic heterocyclic group having 2 to 12 carbon atoms and 1 to 3 heteroatoms, an aromatic heterocyclic group having 3 to 12 carbon atoms and 1 to 3 heteroatoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

Suitable examples of an aliphatic hydrocarbon group are a linear or branched alkyl group, a cycloalkyl group, a linear or branched alkenyl group, a cycloalkenyl group and a linear or branched alkynyl group. Suitable examples of an aliphatic heterocyclic group are a heterocycloalkyl group and a heterocycloalkenyl group. The aromatic heterocyclic group may also be referred to as a heteroaromatic group.

A linear alkyl group as referred to herein has preferably 1 to 30 carbon atoms, more preferably 1 to 24 carbon atoms, and even more preferably 1 to 12 carbon atoms. A branched alkyl group as referred to herein has preferably 3 to 30 carbon atoms, more preferably 3 to 24 carbon atoms, and even more preferably 3 to 12 carbon atoms. A cycloalkyl group as referred to herein has preferably 3 to 30 carbon atoms, more preferably 3 to 24 carbon atoms, and even more preferably 3 to 12 carbon atoms. A linear alkenyl group as referred to herein has preferably 2 to 30 carbon atoms, more preferably 2 to 24 carbon atoms, and even more preferably 2 to 12 carbon atoms. A branched alkenyl group as referred to herein has preferably 4 to 30 carbon atoms, more preferably 4 to 24 carbon atoms, and even more preferably 4 to 12 carbon atoms. A cycloalkenyl group as referred to herein has preferably 3 to 30 carbon atoms, more preferably 3 to 24 carbon atoms, and even more preferably 3 to 12 carbon atoms. A linear alkynyl group as referred to herein has preferably 2 to 30 carbon atoms, more preferably 2 to 24 carbon atoms, and even more preferably 2 to 12 carbon atoms. A branched alkynyl group as referred to herein has preferably 4 to 30 carbon atoms, more preferably 4 to 24 carbon atoms, and even more preferably 4 to 12 carbon atoms. An aromatic hydrocarbon group as referred to herein has preferably 6 to 30 carbon atoms, more preferably 6 to 22 carbon atoms, and even more preferably 6 to 14 carbon atoms. A heterocycloalkyl group as referred to herein has preferably 2 to 30 carbon atoms and 1 to 5 heteroatoms, more preferably 2 to 24 carbon atoms and 1 to 5 heteroatoms, and even more preferably 2 to 12 carbon atoms and 1 to 3 heteroatoms. A heterocycloalkenyl group as referred to herein has preferably 2 to 30 carbon atoms and 1 to 5 heteroatoms, more preferably 2 to 24 carbon atoms and 1 to 5 heteroatoms, and even more preferably 2 to 12 carbon atoms and 1 to 3 heteroatoms. A heteroaromatic hydrocarbon group as referred to herein has preferably 2 to 30 carbon atoms and 1 to 5 heteroatoms, more preferably 3 to 22 carbon atoms and 1 to 5 heteroatoms, and even more preferably 3 to 12 carbon atoms and 1 to 3 heteroatoms. The heteroatom is preferably selected from the group consisting of a nitrogen atom, an oxygen atom and a sulphur atom.

In formula (III), R⁸ and R⁹ are preferably independently selected from the group consisting of hydrogen, a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic hydrocarbon group which may be substituted, and wherein R⁸ and R⁹ may be bonded to each other to form a ring together with the carbon atoms to which they are bound.

The optionally substituted groups may be substituted with a substituent selected from an alkyl group having 1 to 12 carbon atoms, a cycloalkyl group having 3 to 12 carbon atoms, a heterocycloalkyl group having 2 to 12 carbon atoms, an alkenyl group having 2 to 12 carbon atoms, a cycloalkenyl group having 3 to 12 carbon atoms, an alkynyl group having 2 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn carry one or more, such as one, two or three, substituents selected from an alkyl group having 1 to 12 carbon atoms, a cycloalkyl group having 3 to 12 carbon atoms, a heterocycloalkyl group having 2 to 12 carbon atoms, an alkenyl group having 2 to 12 carbon atoms, a cycloalkenyl group having 3 to 12 carbon atoms, an alkynyl group having 2 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

In formula (III), more preferably R⁸ and R⁹ are independently selected from the group consisting of hydrogen, a linear or branched alkyl group which may be substituted, a cycloalkyl group which may be substituted, a heterocycloalkyl group which may be substituted, a linear or branched alkenyl group which may be substituted, a cycloalkenyl group which may be substituted, an aromatic hydrocarbon group which may be substituted, an aralkyl group which may be substituted and a heteroaromatic hydrocarbon group which may be substituted.

The optionally substituted groups may be substituted with a substituent selected from an alkyl group having 1 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn carry one or more, such as one, two or three, substituents selected from an alkyl group having 1 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

### Reactive component

In the process of the present invention, the compound comprising a non-aromatic C-C double bond or C-C triple bond as discussed above is reacted with a reactive component which is obtainable by combining the aminal with an acidic medium comprising trifluoroacetic acid (TFA). Thus, the process in accordance with the invention preferably comprises a further step of preparing a reactive component by combining the aminal with an acidic medium comprising trifluoroacetic acid.

Such a preferred process in accordance with the invention is therefore defined as a process for producing an amine via a hydroaminomethylation reaction of a non-aromatic C-C double bond or C-C triple bond, said process comprising
a step of preparing a reactive component by combining an aminal with an acidic medium comprising trifluoroacetic acid, and
a step of reacting a compound comprising a non-aromatic C-C double bond or C-C triple bond with the reactive component obtained by combining the aminal with the acidic medium comprising trifluoroacetic acid,
wherein the aminal contains two amino groups independently selected from a secondary and a tertiary amino group that are linked via a methylene group, and at least one of the amino groups carries a hydrogen atom at a carbon atom bound in α-position to its nitrogen atom.

The step of preparing the reactive component may be carried out prior to the step of reacting the compound comprising a non-aromatic C-C double bond or C-C triple bond with the reactive component, or concurrently with the step of reacting the compound comprising a non-aromatic C-C double bond or C-C triple bond with the reactive component. Preferably, it is carried out prior to the step of reacting the compound comprising a non-aromatic C-C double bond or C-C triple bond with the reactive component.

Typically, the aminal is combined with the acidic medium simply by mixing the aminal and the acidic medium, with at least the acidic medium being in the form of a liquid. The aminal may be combined with the acidic medium to provide the reactive component e.g. by first providing the acidic medium in a reaction vessel, and adding the aminal, or by first providing the aminal as a first component in a reaction vessel, and adding the acidic medium to the aminal. If the acidic medium comprises more than one component (e.g. TFA and a further organic solvent), the combination of the aminal with the acidic medium may be accomplished in multiple steps, e.g. by first combining the aminal with the organic solvent, and subsequently combining the mixture with the TFA.

The acidic medium with which the aminal is combined to provide the reactive component comprises trifluoroacetic acid (TFA) or consists of TFA.

The acidic medium may be neat TFA. Neat TFA means TFA to which no further compounds have been added. Typically, neat TFA has a purity of at least 95 wt%, preferably at least 98 wt%, and more preferably of at least 99 wt%. Since TFA is a hygroscopic substance, the TFA may comprise water, and it is not necessary to dry it before it is used in the process in accordance with the invention.

In an acidic medium comprising TFA, further components may be contained. Preferably, no other proton donating acids (e.g. acids with a pK_{A} value at 25°C in water of 10 or less) apart from TFA are contained in the acidic medium.

In the acidic medium comprising TFA, the TFA may be present in combination with a further organic solvent. The acidic medium may also consist of TFA and a further organic solvent. Examples of the further organic solvent include benzene, substituted benzene such as chlorbenzene or toluene, dichloroethene (DCE), acetonitrile, ester solvents such as ethyl acetate, dimethylformamide (DMF), dimethylsulfoxide (DMSO), chloroform, dichloromethane (DCM), and alcohols such as methanol, ethanol and isopropanol. The further organic solvent is preferably a polar organic solvent, and more preferably a polar aprotic organic solvent, such as DCE, toluene or acetonitrile. When the acidic medium comprises a further organic solvent, or when it consists of TFA and a further organic solvent, the volume ratio (at 25 °C) of TFA and the further organic solvent in the acidic medium is preferably in the range of 1:5 to 5:1, more preferably in the range of 1:2 to 2:1.

Preferably, the TFA is present in the acidic medium in an amount of 1 molar equivalent or more, more preferably 5 molar equivalents or more, and still more preferably 10 molar equivalents or more, for each mole of C-C double bond or C-C triple bond that is subjected to the hydroaminomethylation reaction. Maximum amounts are not particularly limited. However, too high amounts may not be economic. Typically, amounts of 30 molar equivalents or less are used. The amount of the acidc medium used in the process of the present invention can be adjusted accordingly, depending on the concentration of the TFA in the acidic medium. It will be understood in this regard that the amount of TFA that is present in the reactive component, including the deprotonated form (i.e. a carboxylate anion) of the TFA that may be formed when the acidic medium is combined with the aminal, corresponds to the amount that is present in the acidc medium.

Preferably, to provide the reactive component, the aminal is combined with the acidic medium in an amount of the aminal of 1 molar equivalent or more, more preferably 1.5 molar equivalents or more, and still more preferably 3 molar equivalents or more, for each mole of C-C double bond or C-C triple bond that is subjected to the hydroaminomethylation reaction. Maximum amounts are not particularly limited. However, too high amounts may not be economic. Typically, amounts of 10 molar equivalents or less, preferably 5 molar equivalents or less are used. It will be understood in this regard that the amount of aminal that is present in the reactive component, together with the amount of iminium ions that may be formed when the acidic medium is combined with the aminal, corresponds to the amount that is initially combined with the acidc medium.

While the aminal is combined with the acidic medium, it is advantageous to control the temperature of the mixture wherein the aminal comes into contact with TFA. Preferably, the temperature is controlled to 10 °C or less, more preferably 0 °C or less. As will be understood by the skilled person, the minimum temperature should be higher than the freezing point of the mixture, e.g. - 15 °C or more.

Without wishing to be bound by theory, it is assumed that the TFA of the acidic medium interacts as an acid with the aminal (e.g. (1)) to provide an iminium ion (e.g.(1a)) as a reactive species in the reactive component. Subsequently, a hydroaminomethylation reaction takes place, wherein the iminium ion is added as an electrophile to the non-aromatic double bond (e.g. in compound (2)) or triple bond (e.g. in compound (3)), including a hydride shift from a substituent that was originally bound to the nitrogen atom of the aminal. This sequence is shown in the following reaction scheme 2, which is intended as an illustration of the possible mechanism of the reaction underlying the claimed process, and not as a limitation thereof. The dashed bond indicates the position where a single bond will be present if compound (2) is used as a reactant, and a double bond is present if compound (3) is used as a reactant. The groups R, R^{A} and R^{B} represent any desired atoms or groups, and the aminal, the compound comprising non-aromatic double bond or the compound comprising a triple bond used in the present invention are not limited to the exemplified structures (1), (2) and (3).

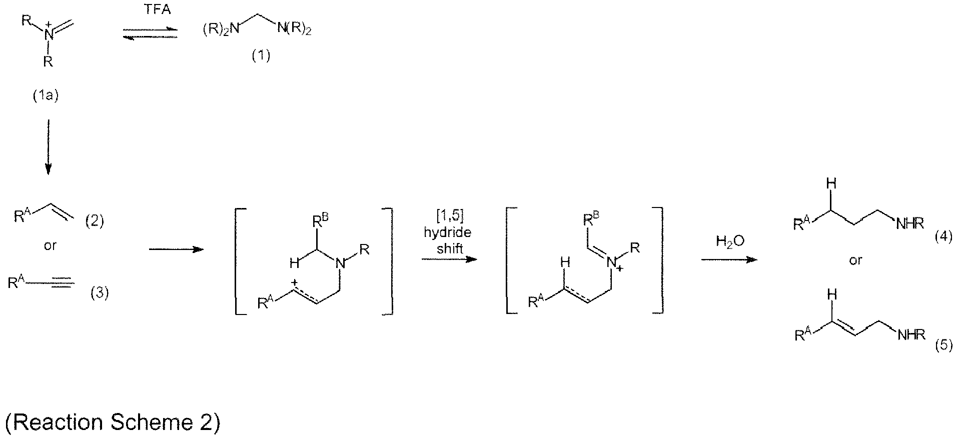

The present inventors surprisingly found that the interaction of the aminal with TFA to provide the reactive component allows the hydroaminomethylation reaction to proceed with an advantageous efficiency, in particular in terms of the yield and the selectivity, compared to reactive species/imminium ions from other sources.

### Hydroaminomethylation Reaction

In the process of the present invention, the compound comprising a non-aromatic C-C double bond or the compound comprising a C-C triple bond is reacted with the reactive component. All the reactants and their preferred embodiments are described in detail above.

The reaction can be initiated e.g. by simply adding the compound comprising a non-aromatic C-C double bond or triple bond to the reactive component, or by adding the reactive component to the compound comprising a non-aromatic C-C double bond or C-C triple bond, and mixing the two. Alternatively, the reactive component may be formed in the presence of the compound comprising a non-aromatic C-C double bond or triple bond, e.g. by mixing the compound comprising a non-aromatic C-C double bond or triple bond in separate steps with the aminal and with the acidic medium, or with the aminal, with TFA, and with any optional consitituents of the acidic medium, such as an organic solvent. If necessary, e.g. if a compound comprising a non-aromatic C-C double bond is used which is in a gaseous state under standard conditions (e.g. 25 °C, 100 kPa), the reaction can be carried out under increased pressure in a suitable apparatus.

Generally, no further components are required to carry out the reaction, and the reactive component which comprises TFA optionally in combination with a further organic solvent can be conveniently used as a reaction medium. However, if considered expedient, a further solvent can be used as an additional reaction medium.

The reaction of the compound comprising a non-aromatic C-C double bond or triple bond with the reactive component is typically carried out in the absence of a metal catalyst or a photocatalyst, preferably in the absence of both a metal catalyst and a photocatalyst. As will be understood by the skilled reader, such catalytically active components do not act as reatctants themselves, but promote the reaction, e.g. by reducing the required activation energy, or, especially in the case of a photocatalyst, by absorbing light and making the energy of the absorbed light available for the reaction that is to be catalyzed.

The reaction temperature for the reaction between the compound comprising a non-aromatic C-C double bond or the compound comprising a C-C triple bond with the reactive component can be suitably adjusted so that the reaction proceeds at an appropriate rate. In fact, the inventors have found that the reaction can be efficiently carried out at temperatures which are lower than those reported for known hydroaminomethylation reactions. For example, the reaction can be carried out at a reaction temperature in the range of 0°C to 150°C, preferably 20°C to 100°C, and more preferably 50°C to 90°C.

A suitable reaction time for the reaction between the compound comprising a non-aromatic C-C double bond or the compound comprising a C-C triple bond with the reactive component is from 10 min to 72 h, preferably from 2 h to 20 h and more preferably from 8 h to 16 h.

Following the reaction of the compound comprising a non-aromatic C-C double bond or triple bond with the reactive component, the process in accordance with the invention allows the structure of the resulting amine to be varied via a further reaction of the product which is obtained from the reaction of the compound comprising a non-aromatic C-C double bond or C-C triple bond with the reactive component. As illustrated in the above reaction scheme 2, it is assumed that the combination of the aminal and the TFA in the reactive component provides an iminium ion as a reactive species, which is added as an electrophile to the non-aromatic double bond or triple bond when the compound comprising a non-aromatic C-C double bond or C-C triple bond is reacted with the reactive component. This addition reaction and a subsequent hydride shift would provide an intermediate product which contains an iminium group.

As a result of the hydroaminomethylation reaction, the C-C double bond is thus converted into a single bond, and the C-C triple bond is converted into a C-C double bond.

The addition of water to the reaction mixture resulting from the reaction of the compound comprising a non-aromatic C-C double bond or triple bond with the reactive component thus results in the formation of a compound containing a secondary amino group, as illustrated in reaction scheme 2. If a compound comprising a C-C double bond is used as a starting material, this secondary amino group is bound via a methylene group to one of the carbon atoms of the former C-C double bond that has been converted into a single bond by the hydroaminomethylation reaction. If a compound comprising a C-C triple bond is used as a starting material, the secondary amino group is typically an allylic amino group, i.e. the former C-C triple bond is converted into a double bond by the hydroaminomethylation reaction, and the secondary amino group is bound via a methylene group to one of the carbon atoms of the former C-C triple bond that has been converted into a double bond.

Thus, in one preferred embodiment, the process in in accordance with the invention further comprises a step wherein water is added to the reaction mixture resulting from the step of reacting a compound comprising a non-aromatic C-C double bond or C-C triple bond with the reactive component.

For example, the water may be conveniently added in the form of a solution of a base in water. Thus, the addition of the water also accomplishes a work-up of the reaction mixture comprising an acid by neutralizing the acid before the product is isolated. For example, the solution of the base can have a concentration of the base between 0.1 mol/l and 4 mol/l, preferably between 0.5 mol/l and 2 mol/l. Examples of the base comprise ammonia, a metal carbonate, a metal hydrogencarbonate and a metal hydroxide. The metal is preferably selected from sodium, potassium, calcium, lithium and magnesium. Preferred examples of the base are sodium hydroxide, potassium hydroxide, calcium carbonate and calcium hydrogencarbonate.

In another preferred embodiment, the process in accordance with the invention further comprises a step wherein an organic nucleophilic reactant is added to the reaction mixture resulting from the step of reacting a compound comprising a non-aromatic C-C double bond or C-C triple bond with the reactive component.

The addition of such a nucleophilic organic reactant allows a tertiary amine to be provided. In this regard, it is assumed that the nucleophilic organic reactant can undergo a direct reaction with an intermediate product which contains an iminium group as illustrated in reaction scheme 2 above. The resulting amine contains a tertiary amino group. If a compound comprising a C-C double bond is used as a starting material, also this tertiary amino group is bound via a methylene group to one of the carbon atoms of the former C-C double bond that has been converted into a single bond by the hydroaminomethylation reaction. If a compound comprising a C-C triple bond is used as a starting material, this tertiary amino group is typically an allylic amino group, i.e. the former C-C triple bond is converted into a double bond by the hydroaminomethylation reaction, and the tertiary amino group is bound via a methylene group to one of the carbon atoms of the former C-C triple bond that has been converted into a double bond.

Examples of suitable nucleophilic reactants include α-CH acidic compounds as they are used as reactants in a Mannich reaction, e.g. ketones with an α-CH group such as acetone or acetophenone. They further include electron-rich aromatic rings or heteroaromatic rings, such as 1,3,5-trimethoxybenzene, indole, pyrrole or benzothiophene. These aromatic or heteroaromatic rings can undergo a Friedel-Crafts-type alkylation reaction with a cationic intermediate product as shown in reaction scheme 2. Following the reaction with the nucleophilic organic reactant, a solution of a base in water can be added before the product is isolated.

In line with the above, in a preferred embodiment the process in accordance with the present invention can be defined as a process for producing an amine via a hydroaminomethylation reaction of a non-aromatic C-C double bond or C-C triple bond, said process comprising
a step of reacting a compound comprising a non-aromatic C-C double bond or C-C triple bond with a reactive component obtainable by combining an aminal with an acidic medium comprising trifluoroacetic acid, and
a step of adding water or a nucleophilic organic reactant to the reaction mixture resulting from the step of reacting a compound comprising a non-aromatic C-C double bond or C-C triple bond with the reactive component,
wherein the aminal contains two amino groups independently selected from a secondary and a tertiary amino group that are linked via a methylene group, and at least one of the amino groups carries a hydrogen atom at a carbon atom bound in α-position to its nitrogen atom.

In a still further preferred embodiment, as indicated above, the process for producing an amine via a hydroaminomethylation reaction of a non-aromatic C-C double bond or C-C triple bond, is thus a process comprising
a step of preparing a reactive component by combining an aminal with an acidic medium comprising trifluoroacetic acid,
a step of reacting a compound comprising a non-aromatic C-C double bond or C-C triple bond with the reactive component obtained by combining the aminal with the acidic medium comprising trifluoroacetic acid, and
a step of adding water to the reaction mixture resulting from the step of reacting a compound comprising a non-aromatic C-C double bond or C-C triple bond with the reactive component, wherein the aminal contains two amino groups independently selected from a secondary and a tertiary amino group that are linked via a methylene group, and at least one of the amino groups carries a hydrogen atom at a carbon atom bound in α-position to its nitrogen atom.

Moreover, conventional steps in amine synthesis reactions can be comprised in the process according to the present invention, such as the protection or the deprotection of an amino group. For example, if an aminal is used in the process in accordance with the invention wherein an amino group is a protected amino group, such as an amino group protected by a benzyl group, an allyl group or a Boc-group, the process of the present invention may further comprise a step of a deprotection of the amino group following the reaction of a compound comprising a non-aromatic C-C double bond or C-C triple bond with the reactive component. The deprotection reaction is not particularly limited and typical deprotection reactions are known in the art.

Alternatively, where desired, it is also possible to protect an amine resulting from the hydroaminomethylation reaction by introducing a protective group for the amine, e,g, a benzyl group, an allyl group or a Boc-group.

Finally, it will be understood that also typical steps for the isolation and/or the purification of a desired product can form part of the process in accordance with the invention, so that the desired product or products can be obtained in high yields and a good quality.

Important aspects of the present invention are summarised in the following items. It will be understood that those items referring back to a preceding item reflect preferred embodiments of the invention.
1. A process for producing an amine via a hydroaminomethylation reaction of a non-aromatic C-C double bond or C-C triple bond, said process comprising
   a step of reacting a compound comprising a non-aromatic C-C double bond or C-C triple bond with a reactive component which is obtainable by combining an aminal with an acidic medium comprising trifluoroacetic acid,
   wherein the aminal contains two amino groups independently selected from a secondary and a tertiary amino group that are linked by a methylene group, and at least one of the amino groups carries a hydrogen atom at a carbon atom bound in α-position to its nitrogen atom.
2. The process according to item 1, which is a process for producing an amine via a hydroaminomethylation reaction of a non-aromatic C-C doule bond, and which comprises a step of reacting a compound comprising a non-aromatic C-C double bond with a reactive component which is obtainable by combining an aminal with an acidic medium comprising trifluoroacetic acid.
3. The process according to item 2, which comprises a step of preparing the reactive component by combining the aminal with the acidic medium comprising trifluoroacetic acid.
4. The process according to item 2 or 3, wherein the acidic medium is neat TFA.
5. The process according to item 2 or 3, wherein the acidic medium comprises a further organic solvent, more preferably selected from the group consisting of benzene which may be substituted, dichloroethene, acetonitrile, ester solvents, dimethylformamide, dimethylsulfoxide, chloroform, dichloromethane, and alcohols.
6. The process according to any of items 2 to 5, wherein the acidic medium comprises TFA in an amount of 1 molar equivalent or more, more preferably 5 molar equivalents or more, and still more preferably 10 molar equivalents or more, for each mole of non-aromatic C-C double bond that is subjected to the hydroaminomethylation reaction.
7. The process according to any of items 2 to 6, wherein the aminal is represented by the following formula (I): 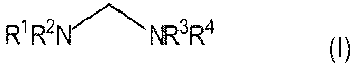 wherein
   R¹ and R³ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted;
   R² and R⁴ are independently selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted;
   and R¹ and R² or R³ and R⁴, respectively, may be bonded to each other to form a ring together with the nitrogen atom to which they are bound, or one of R¹ and R² and one of R³ and R⁴ may be bonded to each other to form a ring together with the methylene group and the nitrogen atoms to which they are bound;
   and wherein at least one of the amino groups -NR¹R² and -NR³R⁴ carries a hydrogen atom at a carbon atom in α-position to the nitrogen atom.
8. The process of item 7, wherein, in formula (I),
   R¹ and R³ are independently selected from the group consisting of hydrogen, a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted;
   R² and R⁴ are independently selected from the group consisting of a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted;
   and R¹ and R² or R³ and R⁴, respectively, may be bonded to each other to form a ring together with the nitrogen atom to which they are bound.
9. The process according to any of items 7 or 8, wherein R¹, R², R³ and R⁴ are independently selected from the group consisting of methyl, ethyl, propyl, isopropyl, butyl, isobutyl, benzyl and allyl.
10. The process according to any of items 7 or 8, wherein R¹ and R² or R³ and R⁴, respectively, are bonded to each other to form a ring together with the nitrogen atom to which they are bound, more preferably a piperidin-1-yl ring.
11. The process according to any of items 7 to 10, wherein R¹, R², R³ and R⁴ are selected such that the groups -NR¹R² and -NR³R⁴ are identical.
12. The process according to any of items 7 to 11, wherein R¹, R², R³ and R⁴ are identical.
13. The process according to any of items 2 to 12, wherein the aminal is combined in an amount of 1 molar equivalent or more, more preferably 1.5 molar equivalents or more, and still more preferably 3 molar equivalents or more, for each mole of non-aromatic C-C double bond that is subjected to the hydroaminomethylation reaction, with the acidic medium.
14. The process according to any of items 2 to 13, wherein the compound comprising a non-aromatic C-C double bond is represented by the following formula (II): 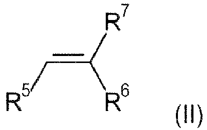 wherein R⁵, R⁶ and R⁷ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, and wherein R⁵ and R⁶ may be bonded to each other to form a ring together with the carbon atoms to which they are bound.
15. The process according to item 14, wherein R⁵, R⁶ and R⁷ are independently selected from the group consisting of hydrogen, a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted, and R⁵ and R⁶ may be bonded to each other to form a ring together with the carbon atoms to which they are bound.
16. The process according to item 14 or 15, wherein
   R⁵ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic hydrocarbon group which may be substituted; and either
   (i) R⁶ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic group which may be substituted; R⁵ and R⁶ may be bonded to each other to form a ring together with the carbon atoms to which they are bound; and R⁷ is selected from the group consisting of hydrogen and methyl, and is more preferably hydrogen; or
   (ii) R⁷ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic group which may be substituted; and R⁶ is selected from the group consisting of hydrogen and methyl, and is more preferably hydrogen.
17. The process according to any of items 2 to 16, wherein said step of reacting the compound comprising a non-aromatic C-C double bond with the reactive component is carried out in the absence of a metal catalyst.
18. The process according to any of items 2 to 17, wherein said step of reacting the compound comprising a non-aromatic C-C double bond with the reactive component is carried out in the absence of a photocatalyst.
19. The process according to any of items 2 to 18, wherein said step of reacting the compound comprising a non-aromatic C-C double bond with the reactive component is carried out at a reaction temperature in the range of 0°C to 150°C, more preferably 20 to 100 °C, still more preferably 50 to 90°C
20. The process according to any of items 2 to 19, wherein the reaction time for the reaction of the compound comprising a non-aromatic C-C double bond with the reactive component ranges from 10 min to 72 h, more preferably from 2 h to 20 h, still more preferably from 8 h to 16 h.
21. The process according to any of items 2 to 20, which further comprises a step wherein water is added to the reaction mixture resulting from the step of reacting a compound comprising a non-aromatic C-C double bond with the reactive component.
22. The process according to any of items 2 to 20, which further comprises a step wherein an organic nucleophilic reactant is added to the reaction mixture resulting from the step of reacting a compound comprising a non-aromatic C-C double bond with the reactive component.
23. The process according to any of items 2 to 22, wherein at least one of the amino groups of the aminal is a protected amino group, and wherein said process further comprises a step of a deprotection of the amino group following the reaction of a compound comprising a non-aromatic C-C double bond.
24. The process according to item 1, which is a process for producing an amine via a hydroaminomethylation reaction of a compound comprising a C-C triple bond, and which comprises a step of reacting a compound comprising a C-C triple bond with a reactive component which is obtainable by combining an aminal with an acidic medium comprising trifluoroacetic acid.
25. The process according to item 24, wherein the process yields an allylic amine.
26. The process according to item 24 or 25, which comprises a step of preparing the reactive component by combining the aminal with the acidic medium comprising trifluoroacetic acid.
27. The process according to any of items 24 to 26, wherein the acidic medium is neat TFA.
28. The process according to item 24 to 26, wherein the acidic medium comprises a further organic solvent, more preferably selected from the group consisting of benzene which may be substituted, dichloroethene, acetonitrile, ester solvents, dimethylformamide, dimethylsulfoxide, chloroform, dichloromethane, and alcohols.
29. The process according to any of items 24 to 28, wherein the acidic medium comprises TFA in an amount of 1 molar equivalent or more, more preferably 5 molar equivalents or more, and still more preferably 10 molar equivalents or more, for each mole of C-C triple bond that is subjected to the hydroaminomethylation reaction.
30. The process according to any of items 24 to 29, wherein the aminal is represented by the following formula (I): 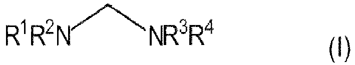 wherein
   R¹ and R³ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted;
   R² and R⁴ are independently selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted;
   and R¹ and R² or R³ and R⁴, respectively, may be bonded to each other to form a ring together with the nitrogen atom to which they are bound, or one of R¹ and R² and one of R³ and R⁴ may be bonded to each other to form a ring together with the methylene group and the nitrogen atoms to which they are bound;
   and wherein at least one of the amino groups -NR¹R² and -NR³R⁴ carries a hydrogen atom at a carbon atom in α-position to the nitrogen atom.
31. The process of item 30, wherein, in formula (I),
   R¹ and R³ are independently selected from the group consisting of hydrogen, a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted;
   R² and R⁴ are independently selected from the group consisting of a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted;
   and R¹ and R² or R³ and R⁴, respectively, may be bonded to each other to form a ring together with the nitrogen atom to which they are bound.
32. The process according to any of items 30 or 31, wherein R¹, R², R³ and R⁴ are independently selected from the group consisting of methyl, ethyl, propyl, isopropyl, butyl, isobutyl, benzyl and allyl.
33. The process according to any of items 30 or 31, wherein R¹ and R² or R³ and R⁴, respectively, are bonded to each other to form a ring together with the nitrogen atom to which they are bound, more preferably a piperidin-1-yl ring.
34. The process according to any of items 30 to 33, wherein R¹, R², R³ and R⁴ are selected such that the groups -NR¹R² and -NR³R⁴ are identical.
35. The process according to any of items 30 to 34, wherein R¹, R², R³ and R⁴ are identical.
36. The process according to any of items 24 to 35, wherein the aminal is combined in an amount of 1 molar equivalent or more, more preferably 1.5 molar equivalents or more, and still more preferably 3 molar equivalents or more, for each mole of C-C triple bond that is subjected to the hydroaminomethylation reaction, with the acidic medium.
37. The process according to any of items 24 to 36, wherein the compound comprising a C-C triple bond is represented by the following formula (III): 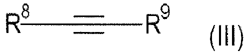
   wherein R⁸ and R⁹ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, and
   wherein R⁸ and R⁹ may be bonded to each other to form a ring together with the carbon atoms to which they are bound.
38. The process according to item 37, wherein R⁸ and R⁹ are independently selected from the group consisting of a hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic hydrocarbon group which may be substituted, and
   wherein R⁸ and R⁹ may be bonded to each other to form a ring together with the carbon atoms to which they are bound.
39. The process according to item 37 or 38, wherein R⁸ and R⁹ are independently selected from the group consisting of a hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic hydrocarbon group which may be substituted.
40. The process according to any of items 24 to 39, wherein said step of reacting the compound comprising a C-C triple bond with the reactive component is carried out in the absence of a metal catalyst.
41. The process according to any of items 24 to 40, wherein said step of reacting the compound comprising a C-C triple bond with the reactive component is carried out in the absence of a photocatalyst.
42. The process according to any of items 24 to 41, wherein said step of reacting the compound comprising a C-C triple bond with the reactive component is carried out at a reaction temperature in the range of 0°C to 150°C, more preferably 20 to 100 °C, still more preferably 50 to 90°C
43. The process according to any of items 24 to 42, wherein the reaction time for the reaction of the compound comprising a C-C triple bond with the reactive component ranges from 10 min to 72 h, more preferably from 2 h to 20 h, still more preferably from 8 h to 16 h.
44. The process according to any of items 24 to 43, which further comprises a step wherein water is added to the reaction mixture resulting from the step of reacting a compound comprising a C-C triple bond with the reactive component.
45. The process according to any of items 24 to 43, which further comprises a step wherein an organic nucleophilic reactant is added to the reaction mixture resulting from the step of reacting a compound comprising a C-C triple bond with the reactive component.
46. The process according to any of items 24 to 45, wherein at least one of the amino groups of the aminal is a protected amino group, and wherein said process further comprises a step of a deprotection of the amino group following the reaction of a compound comprising a C-C triple bond.

### Examples

### General Information

All glassware was oven dried at 100 °C before use. All solvents were distilled from appropriate drying agents prior to use. All reagents were used as received from commercial suppliers unless otherwise stated. Neat infrared spectra were recorded using a Perkin-Elmer Spectrum 100 FT-IR spectrometer. Wavenumbers (v = 1/λ) are reported in cm⁻¹. Mass spectra were obtained using a Finnigan MAT 8200 (70 eV) or an Agilent 5973 (70 eV) spectrometer, using electrospray ionization (ESI) All ¹H NMR and ¹³C NMR experiments were recorded using Bruker AV-400, AV-600 and AV-700 spectrometers at 300 K. Chemical shifts (δ) are quoted in ppm and coupling constants (J) are quoted in Hz. The 7.26 ppm resonance of residual CHCl₃ for proton spectra and 77.16 ppm resonance for carbon spectra were used as internal references. ¹H NMR splitting patterns were designated as singlet (s), doublet (d), triplet (t), quartet (q) or combinations thereof, as well as broad (br). Splitting patterns that could not be interpreted were designated as multiplet (m). Reaction progress was monitored by thin layer chromatography (TLC) performed on aluminum plates coated with kieselgel F254 with 0.2 mm thickness. Visualization was achieved by a combination of ultraviolet light (254 nm) and acidic potassium permanganate. Flash column chromatography was performed using silica gel 60 (230-400 mesh, Merck and co.).

### Procedures for the Synthesis of Starting Materials

### General Procedure for the Synthesis of Aminals

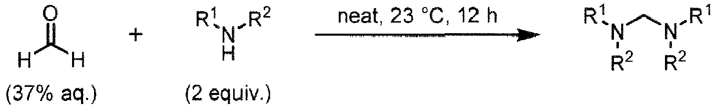

To a round-bottom flask charged with the corresponding free secondary amine (for amines available only as the corresponding salts, 2.00 equiv. of potassium carbonate are added to the reaction mixture) (2.00 equiv.) and a magnetic stir-bar at 0 °C, an aqueous solution of formaldehyde (37%, 1.00 equiv.) was added dropwise and the resulting biphasic mixture was stirred vigorously at ambient temperature (23 °C) for 12 h. The following work-up was dependent on the volatility of the resulting aminal.

Work-up A (for volatile products): Solid potassium hydroxide was added to the reaction mixture until saturation of the aqueous layer was observed. The phases were subsequently separated and the aqueous phase was extracted with diethyl ether (2 x). The organic phases were combined, dried over anhydrous potassium carbonate and filtered. The filtrate was then carefully concentrated under reduced pressure with mild heating, affording the title compound is sufficient purity for further use.

Work-up B (for non-volatile products): The biphasic mixture was separated and the aqueous phase was extracted with ethyl acetate (3 ×). The organic phases were combined, dried over anhydrous sodium sulfate and concentrated under reduced pressure, in most cases affording the title compound in sufficient purity for further use. In the case of incomplete conversion, remaining unreacted amine could be removed by subjection to high vacuum.

### N,N,N',N'-Tetrabutyldiaminomethane (2b)

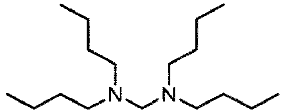

quant.; ¹H NMR (400 MHz, CDCl₃) δ 2.99 (s, 2H), 2.43 (t, J = 7.5 Hz, 8H), 1.42-1.23 (m, 16H), 0.90 (t, *J* = 7.3 Hz, 12H); ¹³C NMR (100 MHz, CDCl₃) δ 75.7, 52.1 (4C), 29.5 (4C), 20.9 (4C), 14.3 (4C); IR (neat) vₘₐₓ: 2955, 2928, 2860, 2799, 2737, 1462, 1375, 1304, 1266, 1240, 1182, 1079; HRMS (ESI+): exact mass calculated for [M+H]⁺ (C₁₇H₃₉N₂) requires *m*/*z* 271.3108, found *m*/*z* 130.1592 (corresponding to *N,N*-dibutylamine).

### N,N,N',N'-Tetrapropyldiaminomethane (2c)

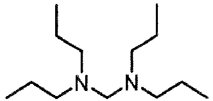

80% yield; used as a crude containing 20% dipropylamine ¹H NMR (400 MHz, CDCl₃) δ 3.01 (s, 2H), 2.42-2.39 (m, 8H), 1.45-1.39 (m, 8H), 0.86 (t, *J* = 7.5 Hz, 12H); ¹³C NMR (100 MHz, CDCl₃) δ 75.7, 54.4 (4C), 20.5 (4C), 12.6 (4C); IR (neat) vₘₐₓ: 2957, 2932, 2872, 2800, 1463, 1377, 1192, 1172; HRMS (ESI+): exact mass calculated for [M+H]⁺ (C₁₃H₃₁N₂) requires *m*/*z* 215.2482, found *m*/*z* 102.1278 (corresponding to *N,N*-dipropylamine).

### N,N,N',N'-Tetraisobutyldiaminomethane (2d)

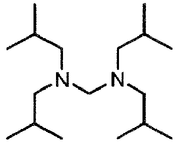

Ca. 30% yield, used as a crude mixture; ¹H NMR (400 MHz, CDCl₃) δ 3.28 (s, 2H), 2.37 (d, *J* = 7.3 Hz, 8H), 1.78-1.70 (m, 4H), 0.90 (d, *J* = 6.6 Hz, 24H).

### N,N,N',N'-Tetrabenzyldiaminomethane (2e)

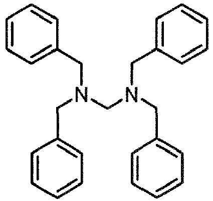

92% yield; ¹H NMR (400 MHz, CDCl₃) δ 7.33-7.26 (m, 16H), 7.26-7.22 (m, 4H), 3.63 (s, 8H), 3.11 (s, 2H); ¹³C NMR (100 MHz, CDCl₃) δ 139.9 (4C), 129.1 (8C), 128.3 (8C), 126.9 (4C), 72.4, 56.3 (4C); IR (neat) vₘₐₓ: 3060, 3026, 2927, 2795, 1493, 1451, 1245, 1125, 1072, 1028, 974, 914; HRMS (ESI+): exact mass calculated for [M+H]⁺ (C₂₉H₃₁N₂) requires *m*/*z* 407.2482, found *m*/*z* 198.1281 (corresponding to *N,N*-dibenzylamine).

### N,N,N',N'-Tetraallyldiaminomethane (2f)

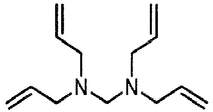

70% yield; ¹H NMR (400 MHz, CDCl₃) δ 5.89-5.77 (m, 4H), 5.17-5.06 (m, 8H), 3.15 (dt, *J* = 6.4, 1.3 Hz, 8H), 3.11 (s, 2H); ¹³C NMR (100 MHz, CDCl₃) δ 136.4 (4C), 116.8 (4C), 72.5, 54.7 (4C); IR (neat) vₘₐₓ: 3077, 2978, 2920, 2801, 1642, 1446, 1417, 1399, 1350, 1259, 1159, 994, 913; HRMS (ESI+): exact mass calculated for [M+H]⁺ (C₁₃H₂₃N₂) requires *m*/*z* 207.1865, found *m*/*z* 98.0694 (corresponding to *N,N*-diallylamine).

### N,N,N',N'-Tetrakis(methyl-d₃)diaminomethane (2a-d₁₂)

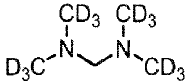

yield not determined-isolated as a -30 mol% solution in diethyl ether and used without further purification; ¹H NMR (600 MHz, CDCl₃) δ 2.69 (s, 2H);

### N,N'-Dibutyl-N,N'-bis(butyl-d₉)diaminomethane (2b-d₁₈)

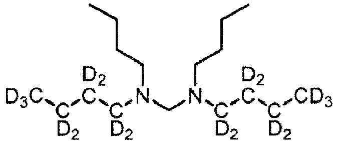

87% yield; ¹H NMR (400 MHz, CDCl₃) δ 2.99 (s, 2H), 2.43 (t, *J* = 7.5 Hz, 4H), 1.42-1.24 (m, 8H), 0.90 (t, *J* = 7.2 Hz, 6H); ¹³C NMR (100 MHz, CDCl₃) δ 75.6, 52.0 (2C), 51.1 (t, *J* = 20.3 Hz), 29.6 (2C), 20.9 (2C), 14.3 (2C), 13.0 (t, *J* = 19.1 Hz)-two signals could not be identified; IR (neat) vₘₐₓ: 2956, 2929, 2861, 2215, 1463, 1376, 1190, 1057; HRMS (ESI+): exact mass calculated for [M+H]⁺ (C₁₇H₂₁D₁₈N₂) requires *m*/*z* 289.4238, found *m*/*z* 139.2156 (corresponding to *N*-butylbutan-d₉-1-amine).

### Di(piperidin-1-yl)methane (2g)

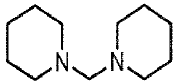

92% yield; ¹H NMR (400 MHz, CDCl₃) δ 2.83 (s, 2H), 2.40 (t, *J* = 5.5 Hz, 8H), 1.54 (pent, *J* = 5.5 Hz, 8H), 1.46-1.39 (m, 4H).

### Synthesis of Alkene Substrates

### 1-(1-Pyrrolidinyl)-10-undecen-1-one (1a)

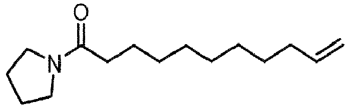

To a solution of pyrrolidine (0.452 mL, 0.391 g, 5.5 mmol, 1.1 equiv.) and triethylamine (1.39 mL, 1.012 g, 10 mmol, 2.00 equiv.) in DCM (20 mL) at 0 °C, 10-undecenoyl chloride (1.08 mL, 1.014 g, 5 mmol, 1 equiv.) was added dropwise and the resulting reaction mixture was allowed to warm to room temperature while stirring overnight (14 h). After this time, a saturated aqueous solution of sodium bicarbonate was added and the biphasic system was separated. The aqueous phase was extracted with DCM (1 x) and the organic phases were combined and dried over anhydrous sodium sulfate. The dried solution was filtered and concentrated under reduced pressure. The resulting crude material was purified by flash column chromatography on silica gel (heptane/ethyl acetate) to afford the desired compound; Quant.; ¹H NMR (400 MHz, CDCl₃) δ 5.80 (ddt, *J* = 16.9, 10.2, 6.7 Hz, 1H), 4.97 (ddd, *J* = 17.1, 3.7, 1.6 Hz, 1H), 4.91 (ddt, *J* = 10.2, 2.3, 1.2 Hz, 1H), 3.45 (t, *J* = 6.9 Hz, 2H), 3.39 (t, *J* = 6.8 Hz, 2H), 2.25-2.03 (m, 2H), 2.01-1.99 (m, 2H), 1.97-1.95 (m, 2H), 1.87-1.80 (m, 2H), 1.65-1.59 (m, 2H), 1.39-1.22 (m, 10H).

### Undec-10-en-1-yl acetate (1w)

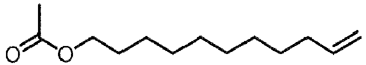

To a solution of 10-undecen-1-ol (0.50 g, 3.20 mmol, 1.00 equiv.) in pyridine (10 mL) was added acetic anhydride (0.91 mL, 9.60 mmol, 3.00 equiv.). After stirring for 3 h at 60 °C, the reaction mixture was diluted with ethyl acetate (25 mL) and washed sequentially with 1 N HCI (25 mL x 5) and brine (25 mL). The organic layer was dried over anhydrous magnesium sulfate, filtered and the filtrate was concentrated. The crude material was purified by flash column chromatography on silica gel (pentane/ethyl acetate) to afford the title compound. 75% yield; ¹H NMR (600 MHz, CDCl₃) δ 5.83-5.78 (m, 1H), 5.00-4.91 (m, 2H), 2.04 (s, 3H), 1.62-1.59 (m, 2H), 1.38-1.27 (m, 14H).

### Diethyl oct-7-en-1-ylphosphonate (1y)

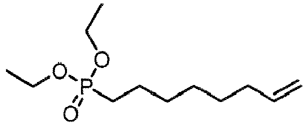

To a round-bottomed flask under Ar atmosphere, sodium hydride (60% dispersion in mineral oil, 520 mg, 13.0 mmol, 1.30 equiv.), anhydrous THF (70 mL), and diethyl phosphite (1.80 g, 13.0 mmol, 1.30 equiv.) were added. After stirring at 0 °C for 0.5 h, and subsequently at reflux (66 °C) for 1.5 h, 8-bromo-1-octene (1.91 g, 10.0 mmol, 1.00 equiv.) was added at 0 °C. After stirring at ambient temperature (23 °C) for 24 h, water (50 mL) was added. The phases were separated and the aqueous phase was extracted with dichloromethane (3 x 50 mL). The organic phases were dried over anhydrous magnesium sulfate, filtered and concentrated. The crude residue was purified by flash column chromatography over silica gel (n-heptane/ethyl acetate). 90% yield; ¹H NMR (600 MHz, CDCl₃) δ 5.80 (ddt, *J* = 16.9, 10.2, 6.7 Hz, 1H), 5.01-4.91 (m, 2H), 4.15-4.04 (m, 4H), 2.06-2.01 (m, 2H), 1.76-1.68 (m, 2H), 1.68-1.54 (m, 2H), 1.40-1.24 (m, 11H).

### N-(Pyridin-4-yl)undec-10-enamide (1z)

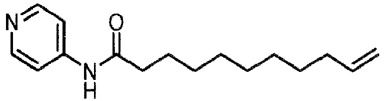

To a mixture of pyridin-4-amine (471 mg, 5.00 mmol, 1.00 equiv.) and triethylamine (1.50 mL, 11.0 mmol, 2.20 equiv.) in DCM (20 mL) was added 10-undecenoylchloride (1.00 mL, 5.00 mmol, 1.00 equiv.) at 0 °C. The mixture was stirred for 12 h at ambient temperature (23 °C), after which aqueous HCI (1 M, 10 mL) was added. The phases were separated and the aqueous phase was extracted with DCM (3 × 15 mL). The combined organic phases were dried over anhydrous sodium sulfate, filtered and subsequently concentrated under reduced pressure. Flash column chromatography on silica gel (heptane/ethyl acetate) of the resulting crude material afforded the title compound. 85% yield; ¹H NMR (600 MHz, CDCl₃) δ 8.51 (dd, *J* = 4.8, 1.5 Hz, 2H), 7.66 (s, 1H), 7.51 (dd, *J* = 4.8, 1.5 Hz, 2H), 5.82 (ddt, *J* = 16.9, 10.2, 6.7 Hz, 1H), 5.01 (ddd, *J* = 17.1, 3.5, 1.5 Hz, 1H), 4.96-4.94 (m, 1H), 2.42-2.39 (m, 2H), 2.07-2.04 (m, 2H), 1.74-1.73 (m, 2H), 1.39-1.28 (m, 10H); ¹³C NMR (150 MHz, CDCl₃) δ 172.3, 150.7, 150.6, 145.3, 139.3, 114.3, 113.6, 113.6, 38.0, 33.9, 29.4 (2C), 29.3, 29.2, 29.0, 25.4; ; IR (neat) vₘₐₓ: 2925, 2854, 1707, 1683, 1592, 1517, 1415, 1329, 1294, 1210, 999; HRMS (ESI+): exact mass calculated for [M+H]⁺ (C₁₆H₂₅N₂O) requires *m*/*z* 261.1961, found *m*/*z* 261.1958.

### Dodec-11-enenitrile (1aa)

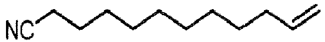

A mixture of 9-decen-1-ol (0.892 mL, 0.781 g, 5 mmol, 1 equiv.), triethylamine (0.767 mL, 0.557 g, 5.5 mmol, 1.5 equiv.), and p-toluenesulfonyl chloride (0.953 g, 5 mmol, 1 equiv.) in DCM (20 mL) was stirred for 12 h at ambient temperature (23 °C). After this time, the reaction mixture was diluted with water (20 mL) and extracted with chloroform (3 × 20 mL). The combined organic phases were washed with a saturated aqueous solution of ammonium chloride, and subsequently dried over anhydrous magnesium sulfate. After removal of the solvent under reduced pressure, potassium cyanide (0.358 g, 5.5 mmol, 1.1 equiv.) and DMSO (30 mL) were added and the resulting suspension was stirred at ambient temperature (23 °C) for 14 h. After this time, water (100 mL) was added and the resulting solution was extracted with diethyl ether (4 × 30 mL). The combined organic phases were washed with a saturated aqueous solution of sodium bicarbonate (70 mL) and subsequently dried over anhydrous magnesium sulfate. The dried solution was filtered and the filtrate was concentrated under reduced pressure to afford the crude product. The crude material was purified by flash column chromatography on silica gel (heptane/ethyl acetate) to afford the title compound. 67% yield; ¹H NMR (600 MHz, CDCl₃) δ 5.86-5.80 (m, 1H), 5.03-4.95 (m, 2H), 2.37-2.35 (t, *J* = 7.8 Hz, 2H), 2.08-2.04 (q, *J* = 7.2 Hz, 2H), 1.70-1.65 (q, *J* = 6.6 Hz, 2H), 1.47-1.45 (m, 2H), 1.40-1.38 (m, 2H), 1.28 (m, 8H).

### N-Butylundec-10-en-1-amine (1ad)

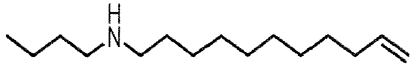

To a stirred solution of 10-undecenal (505 mg, 3.00 mmol, 1.00 equiv.) in MeOH (3 mL) was added n-butylamine (594 µL, 6.00 mmol, 2.00 equiv.), and the mixture was stirred at ambient temperature (23 °C) for 2 h. After this time, the reaction mixture was cooled to 0 °C and sodium borohydride (57.0 mg, 1.50 mmol, 0.75 equiv.) was added. The resulting mixture was stirred at 0 °C for 30 min, after which excess reductant was quenched by the addition of water (3 mL). The crude mixture was extracted with DCM (3 × 5 mL), the combined organic phases were dried over anhydrous magnesium sulfate and the dried solution was filtered. The filtrate was concentrated under reduced pressure and the resulting crude material was purified by flash column chromatography on silica gel (dichloromethane/MeOH/NH₄OH) to afford the title compound. 66% yield; ¹H NMR (600 MHz, CDCl₃) δ 5.81 (ddt, *J* = 16.9, 10.2, 6.7 Hz, 1H), 4.99 (dd, *J* = 17.1, 1.4 Hz, 1H), 4.93-4.90 (m, 1H), 2.67 (ddd, *J* = 12.7, 8.0, 3.0 Hz, 3H), 2.03 (q, *J* = 7.0 Hz, 2H), 1.67-1.51 (m, 3H), 1.43-1.20 (m, 15H), 0.92 (t, *J* = 7.3 Hz, 3H).

### N,N-Diethylundec-10-en-1-amine (1ae)

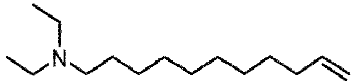

To a stirred solution of 10-undecenal (505 mg, 3.00 mmol, 1.00 equiv.) in MeOH (3 mL) was added *N,N*-diethylamine (621 µL, 6.00 mmol, 2.00 equiv.), and the mixture was stirred at ambient temperature (23 °C) for 2 h. After this time, the reaction mixture was cooled to 0 °C and sodium borohydride (57.0 mg, 1.50 mmol, 0.75 equiv.) was added. The resulting mixture was stirred at 0 °C for 30 min, after which excess reductant was quenched by the addition of water (3 mL). The crude mixture was extracted with DCM (3 × 5 mL), the combined organic phases were dried over anhydrous magnesium sulfate and the dried solution was filtered. The filtrate was concentrated under reduced pressure and the resulting crude material was purified by flash column chromatography on silica gel (dichloromethane/MeOH/NH₄OH) to afford the title compound. 30% yield. ¹H NMR (600 MHz, CDCl₃) δ 5.81 (ddt, *J* = 16.9, 10.2, 6.7 Hz, 1H), 4.99 (ddd, *J* = 17.1, 3.6, 1.6 Hz, 1H), 4.93-4.91 (m, 1H), 2.51 (q, *J* = 7.2 Hz, 4H), 2.40-2.38 (m, 2H), 2.05-2.01 (m, 2H), 1.44-1.42 (m, 2H), 1.38-1.36 (m, 2H), 1.27-1.24 (m, 10H), 1.01 (t, *J* = 7.2 Hz, 6H); ¹³C NMR (150 MHz, CDCl₃) δ 139.4, 114.2, 53.2, 47.0 (2C), 34.0, 29.8, 29.7, 29.6, 29.3, 29.1, 27.9, 27.2, 11.8 (2C); IR (neat) vₘₐₓ: 2968, 2923, 2853, 2797, 1464, 1380, 1202, 1070, 991, 908; HRMS (ESI+): exact mass calculated for [M+H]⁺ (C₁₅H₃₂N) requires *m*/*z* 226.2529, found *m*/*z* 226.2527.

### General Procedures for Redox-Neutral Hydroaminomethylation

### General Procedure A for Hydroaminomethylation of Alkenes and Alkynes

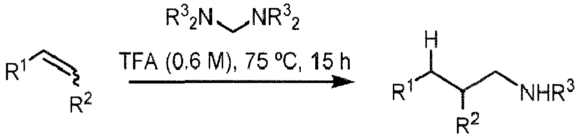

A round-bottom flask charged with *N,N,N',N'*-tetraalkyldiaminomethane (4 equiv. with respect to the alkene or the alkyne) and a magnetic stir-bar under argon-atmosphere was cooled to 0 °C. After this, trifluoroacetic acid (neat TFA, 22.4 equiv. with respect to the alkene or alkyne) was added slowly, maintaining the low temperature of the contents of the flask. After completed addition of TFA, the alkene or alkyne was added in one portion in a concentration of 0.5 mmol (to yield a concentration of 0.6 M of alkene or alkyne in TFA),the flask was sealed and placed in an oil bath at 75 °C. The reaction was vigorously stirred at this temperature for 15 h, after which it was allowed to cool to room temperature. Subsequently, volatile components were removed under reduced pressure. The crude mixture was then treated with aqueous sodium hydroxide (1 M-2 mL/1 mmol substrate) and chloroform (1 mL/1 mmol substrate) and stirred vigorously at room temperature for 1 h. After this time, aqueous sodium hydroxide (5 M) was added until the reaction mixture reaches pH 12. The resulting biphasic mixture was separated and the aqueous phase was extracted with chloroform (3 x 200 mL). The combined organic phases were then dried over anhydrous sodium sulfate and filtered. The filtrate was concentrated under reduced pressure to afford the crude product, which was purified by flash column chromatography on silica gel (dichloromethane/MeOH/NH₄OH 19:1:0.15) to afford the analytically pure desired product.

### General Procedure B for Hydroaminomethylation of Alkynes

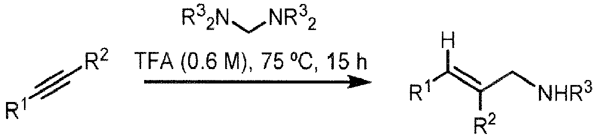

A round-bottom flask charged with *N,N,N',N'*-tetraalkyldiaminomethane (4 equiv.) and a magnetic stir-bar under argon-atmosphere was cooled to 0 °C and dissolved in DCE (1.2 M with respect to the alkyne). After this, trifluoroacetic acid (11.2 equiv. with respect to the alkyne) was added slowly, maintaining the low temperature of the contents of the flask. After completed addition of TFA, the alkyne (0.5 mmol, a concentration of 0.6 M of alkyne in the combined amounts of TFA and DCE) was added in one portion, the flask was sealed and placed in an oil bath at 75 °C. The reaction was vigorously stirred at this temperature for 15 h, after which it was allowed to cool to room temperature. Subsequently, volatile components were removed under reduced pressure. After this time, aqueous sodium hydroxide (1 M) was added until the reaction mixture reaches pH 12 and the mixture was extracted with chloroform (3 x 200 mL). The combined organic phases were then dried over anhydrous sodium sulfate and filtered. The filtrate was concentrated under reduced pressure to afford the crude product, which was purified by flash column chromatography on silica gel (dichloromethane/MeOH/NH₄OH 19:1:0.15) to afford the analytically pure desired product.

### General Procedure C for Hydroaminomethylation of Alkynes

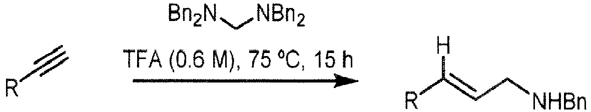

A round-bottom flask charged with *N,N,N',N'*-tetrabenzyldiaminomethane (1.5 equiv.) and a magnetic stir-bar under argon-atmosphere is cooled to 0 °C and dissolved in DCE (1.2 M with respect to the alkyne) slowly, maintaining the low temperature of the contents of the flask. After this, trifluoroacetic acid (11.2 equiv. with respect to the alkyne) was added slowly, maintaining the low temperature of the contents of the flask. After completed addition of TFA, the alkyne (0.5 mmol, to yield a concentration of 0.6 M of alkyne in the combined amounts of TFA and DCE) was added in one portion, the flask was sealed and placed in an oil bath at 75 °C. The reaction was vigorously stirred at this temperature for 15 h, after which it is allowed to cool to room temperature. Subsequently, volatile components were removed under reduced pressure. After this time, aqueous sodium hydroxide (1 M) was added until the reaction mixture reaches pH 12 and the mixture was extracted with chloroform (3 x 200 mL). The combined organic phases were then dried over anhydrous sodium sulfate and filtered. The filtrate was concentrated under reduced pressure to afford the crude product, which was purified by flash column chromatography on silica gel (dichloromethane/MeOH/NH₄OH 19:1:0.15) to afford the analytically pure desired product.

### Example 1: 12-(Methylamino)-1-(pyrrolidin-1-yl)dodecan-1-one (3a)

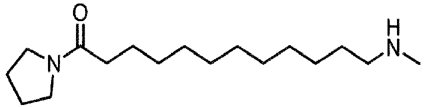

Prepared with 4 equiv. *N,N,N',N'*-tetramethyldiaminomethane according to general procedure A; 84% yield; ¹H NMR (400 MHz, CDCl₃) δ 3.45 (t, *J* = 6.8 Hz, 2H), 3.40 (t, *J* = 6.9 Hz, 2H), 2.54 (t, *J* = 7.2 Hz, 2H), 2.42 (s, 3H), 2.24 (t, *J* = 7.8 Hz, 2H), 1.93 (app quin, *J* = 6.6 Hz, 2H), 1.83 (app quin, *J* = 6.6 Hz, 2H), 1.63 (app quin, *J* = 7.3 Hz, 2H), 1.46 (app quin, *J* = 6.9 Hz, 2H), 1.35-1.22 (m, 14H), 1.07 (br s, 1H); ¹³C NMR (100 MHz, CDCl₃) δ 171.9, 52.4, 46.7, 45.7, 36.7, 35.0, 30.1, 29.7 (2C), 29.7 (2C), 29.6, 29.6, 27.5, 26.3, 25.1, 24.6; IR (neat) vₘₐₓ: 3439, 2924, 2852, 1636, 1558, 1439, 1382, 1345, 1310, 1254, 1227; HRMS (ESI+): exact mass calculated for [M+H]⁺ (C₁₇H₃₅N₂O) requires *m*/*z* 283.2744, found *m*/*z* 283.2743.

### Example 2: 12-(Butylamino)-1-(pyrrolidin-1-yl)dodecan-1-one (3b)

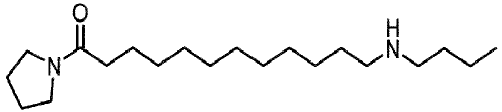

Prepared with 4 equiv. *N,N,N',N'-*tetrabutyldiaminomethane according to general procedure A; 85% yield; ¹H NMR (400 MHz, CDCl₃) δ 3.45 (t, *J* = 6.8 Hz, 2H), 3.40 (t, *J* = 6.9 Hz, 2H), 2.61-2.54 (m, 4H), 2.24 (t, *J* = 7.8 Hz, 2H), 1.98-1.90 (m, 2H), 1.87-1.80 (m, 2H), 1.63 (app quin, *J* = 7.3 Hz, 2H), 1.51-1.41 (m, 4H), 1.37-1.23 (m, 16H), 1.06 (br s, 1H), 0.91 (t, *J =* 7.4 Hz, 3H); ¹³C NMR (100 MHz, CDCl₃) δ 172.0, 50.3, 50.0, 46.7, 45.7, 35.0, 32.5, 30.4, 29.7 (2C), 29.7 (2C), 29.6, 29.6, 27.6, 26.3, 25.1, 24.6, 20.7, 14.2; IR (neat) vₘₐₓ: 2924, 2854, 1643, 1432, 1345; HRMS (ESI+): exact mass calculated for [M+H]⁺ (C₂₀H₄₁N₂O) requires *m*/*z* 325.3213, found *m*/*z* 325.3214.

### Example 3: 12-(Propylamino)-1-(pyrrolidin-1-yl)dodecan-1-one (3c)

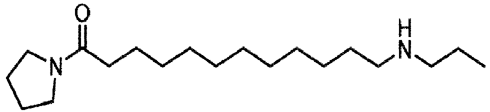

Prepared with 4 equiv. *N,N,N',N'*-tetrapropyldiaminomethane according to general procedure A; 92% yield; ¹H NMR (600 MHz, CDCl₃) δ 3.45 (t, *J* = 6.8 Hz, 2H), 3.40 (t, *J* = 6.9 Hz, 2H), 2.54 (app dt, *J* = 11.7, 7.3 Hz, 4H), 2.23 (t, *J* = 7.8 Hz, 2H), 1.93 (app quin, *J* = 6.8 Hz, 2H), 1.83 (app quin, *J* = 6.8 Hz, 2H), 1.63 (app quin, *J* = 7.4 Hz, 2H), 1.53-1.42 (m, 4H), 1.35-1.20 (m, 15H), 0.90 (t, *J* = 7.4 Hz, 3H); ¹³C NMR (150 MHz, CDCl₃) δ 171.9, 52.4, 50.3, 46.7, 45.7, 35.0, 30.4, 29.7 (2C), 29.7, 29.7, 29.6, 29.6, 27.6, 26.3, 25.1, 24.6, 23.4, 12.0; IR (neat) vₘₐₓ: 2924, 2853, 1641, 1432, 1343; HRMS (ESI+): exact mass calculated for [M+H]⁺ (C₁₉H₃₉N₂O) requires *m*/*z* 311.3057, found *m*/*z* 311.3058.

### Example 4: 11-(Isobutylamino)-1-(pyrrolidin-1-yl)undecan-1-one (3d)

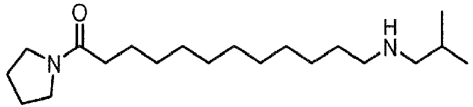

Prepared with 4 equiv. *N,N,N',N'*-tetraisobutyldiaminomethane according to general procedure A, 84% yield; ¹H NMR (600 MHz, CDCl₃) δ 3.45 (t, *J* = 6.9 Hz, 2H), 3.40 (t, *J* = 6.8 Hz, 2H), 2.58-2.56 (m, 2H), 2.41 (d, *J* = 6.8 Hz, 2H), 2.25-2.23 (m, 2H), 1.93 (dd, *J* = 13.5, 6.8 Hz, 2H), 1.85 (dd, *J* = 13.7, 6.8 Hz, 2H), 1.75 (m, 1H), 1.63 (dt, *J* = 15.1, 7.5 Hz, 2H), 1.49-1.46 (m, 2H), 1.35-1.22 (m, 13H), 0.90 (d, *J* = 6.6 Hz, 6H); ¹³C NMR (150 MHz, CDCl₃) δ 172.0, 58.3, 50.3, 46.8, 45.7, 35.0, 30.2, 29.7 (2C), 29.7 (2C), 29.6, 29.6, 28.4, 27.5, 26.3, 25.1, 24.7, 20.9 (2C) ppm; IR (neat) vₘₐₓ: 2922, 2852, 2808, 1641, 1429, 1365, 1194, 1127; HRMS (ESI+): exact mass calculated for [M+H]⁺(C₂₀H₄₁N₂O) requires *m*/*z* = 325.3213, found *m*/*z* 325.3209.

### Example 5: 12-(Benzylamino)-1-(pyrrolidin-1-yl)dodecan-1-one (3e)

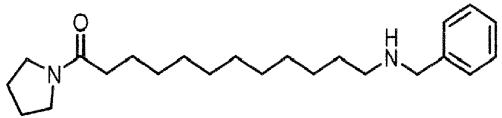

Prepared with 1.5 equiv. *N,N,N',N'*-tetrabenzyldiaminomethane according to general procedure A, 86% yield. ¹H NMR (400 MHz, CDCl₃) δ 7.27-7.25 (m, 4H), 7.21-7.15 (m, 1H), 3.73 (s, 2H), 3.39 (t, *J* = 6.9 Hz, 2H), 3.33 (t, *J* = 6.8 Hz, 2H), 2.58-2.54 (m, 2H), 2.20-2.16 (m, 2H), 1.98 (br s, 1H), 1.88-1.75 (m, 2H), 1.79-1.75 (m, 2H), 1.59-1.56 (m, 2H), 1.47-1.43 (m, 2H), 1.22 (m, 15H); ¹³C NMR (100 MHz, CDCI3) δ 171.8, 140.1, 128.4 (2C), 128.2 (2C), 54.0, 53.2, 49.4, 46.6, 45.6, 34.8, 29.9, 29.5 (2C), 29.5 (2C), 29.5, 29.4, 27.3, 26.1, 24.9, 26.4; IR (neat) vₘₐₓ: 3061, 3026, 2917, 2812, 1602, 1494, 1453, 1115, 734, 697; HRMS (ESI+): exact mass calculated for [M+H]⁺ (C₂₃H₃₉N₂O) requires *m*/*z* 359.3057, found *m*/*z* 359.3056.

### Example 6: 12-(Allylamino)-1-(pyrrolidin-1-yl)dodecan-1-one (3f)

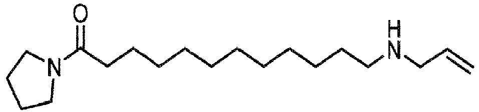

Prepared with 4 equiv. *N,N,N',N'*-tetraallyldiaminomethane according to general procedure A, 86% yield. ¹H NMR (400 MHz, CDCl₃) δ 5.96-5.86 (m, 1H), 5.12 (dddd, *J* = 32.1, 10.2, 3.2, 1.4 Hz, 2H), 3.43 (dt, *J* = 21.7, 6.9 Hz, 4H), 3.24 (dt, *J* = 6.0, 1.4 Hz, 2H), 2.61-2.58 (m, 2H), 2.26-2.22 (m, 2H), 1.96-1.91 (m, 2H), 1.88-1.83 (m, 2H), 1.66-1.60 (m, 2H), 1.50-1.44 (m, 2H), 1.30-1.27 (m, 14H), 0.84 (br s, 1H); ¹³C NMR (100 MHz, CDCl₃) δ 172.0, 137.3, 115.7, 52.8, 49.7, 46.8, 45.7, 35.0, 30.3, 29.7 (2C), 29.7 (2C), 29.6, 29.6, 27.5, 26.3, 25.1, 24.6.; IR (neat) vₘₐₓ: 2922, 2852, 1641, 1432, 1168, 1033, 994, 914; HRMS (ESI+): exact mass calculated for [M+H]⁺ (C₁₉H₃₇N₂O) requires *m*/*z* 309.2900, found *m*/*z* 309.2928.

### Example 7: 12-Amino-1-(pyrrolidin-1-yl)dodecan-1-one (4f)

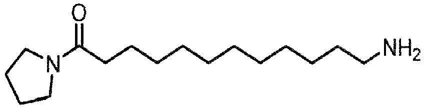

The *N*-allyl compound (**3f**, 0.095 mmol, 29.3 mg) was dissolved in 2 ml ethanol and palladium on charcoal (10%, 0.5 equivalents, 5 mg) was added. The mixture was refluxed for 24h. The solid was filtered off over celite, the ethanol was removed at reduced pressure and the crude residue was purified by flash column chromatography over silica gel (dichloromethane/MeOH/NH₄OH 18/1/0.15) to afford the title compound.
86% yield. ¹H NMR (600 MHz, CDCl₃) δ 3.45 (t, *J* = 6.9 Hz, 2H), 3.40 (t, *J* = 6.8 Hz, 2H), 2.66 (t, *J* = 7.1 Hz, 1H), 2.30-2.18 (m, 2H), 1.93 (p, *J* = 6.8 Hz, 2H), 1.84 (p, *J* = 6.9 Hz, 2H), 1.64-1.61 (m, 2H), 1.47-1.25 (m, 17H); ¹³C NMR (150 MHz, CDCl₃) δ 172.0, 46.7, 45.7, 42.4, 35.0, 34.0, 29.7, 29.7, 29.6, 29.6, 27.0, 26.3, 25.1, 24.6; IR (neat) vₘₐₓ: 3415, 2922, 2851, 1630, 1430, 1342, 1008; HRMS (ESI+): exact mass calculated for [M+H]⁺ (C₁₆H₃₃NO) requires *m*/*z* 269.2587, found *m*/*z* 269.2590.

### Example 8: N-Methyldodecan-1-amine (3g)

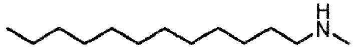

Prepared with 4 equiv. *N,N,N',N'*-tetramethyldiaminomethane according to general procedure A; 61% yield; ¹H NMR (400 MHz, CDCl₃) δ 2.54 (t, *J* = 7.3 Hz, 2H), 2.42 (s, 3H), 1.46 (app quin, *J* = 7.1 Hz, 2H), 1.32-1.21 (m, 18H), 1.10 (br s, 1H), 0.87 (t, *J* = 7.0 Hz, 3H); ¹³C NMR (100 MHz, CDCl₃) δ 52.4, 36.8, 32.1, 30.1, 29.8, 29.8, 29.7 (2C), 29.7, 29.5, 27.5, 22.8, 14.3; IR (neat) vₘₐₓ: 2955, 2921, 2852, 1465, 1380, 1308, 722; HRMS (ESI+): exact mass calculated for [M+H]⁺ (C₁₃H₃₀N) requires *m*/*z* 200.2373, found *m*/*z* 200.2367.

### Example 9: N-Benzyl-3,4,4-trimethylpentan-1-amine (3h)

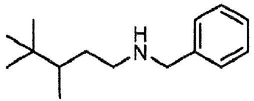

Prepared with 1.5 equiv. *N,N,N',N'*-tetrabenzyldiaminomethane according to general procedure A; 52% yield. ¹H NMR (400 MHz, CDCl₃) δ 7.36-7.27 (m, 5H), 3.87-3.79 (m, 2H), 2.75 (ddd, *J* = 10.9, 9.8, 4.5 Hz, 1H), 2.59 (ddd, *J* = 11.2, 8.8, 6.6 Hz, 1H), 1.80-1.73 (m, 1H), 1.65 (br s, 1H, NH), 1.28-1.14 (m, 2H), 0.87 (s, 9H), 0.85 (d, *J* = 6.5 Hz, 3H) ppm; ¹³C NMR (100 MHz, CDCl₃) δ 140.5, 128.5 (2C), 128.3 (2C), 127.1, 54.3, 49.0, 41.1, 33.1, 32.2, 27.4 (3C), 14.6 ppm; IR (neat) vₘₐₓ: 2958, 2865, 2831, 1453, 1364, 1119, 1028, 732, 697; HRMS (ESI+): exact mass calculated for [M+H]⁺ (C₁₅H₂₆N) requires *m*/*z* 220.2060, found *m*/*z* 220.2057.

### Example 10: tert-Butyl (cycloheptylmethyl)(methyl)carbamate (3i)

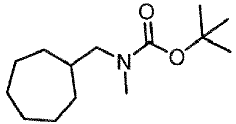

Prepared with 4 equiv. *N,N,N',N'*-tetramethyldiaminomethane according to general procedure A followed by the protection of the amino group by a Boc protective group; 78% yield; ¹H NMR (400 MHz, CDCl₃) δ 3.01-2.99 (m, 2H), 2.81 (s, 3H), 1.82-1.72 (m, 1H), 1.70-1.46 (m, 8H), 1.45 (s, 9H), 1.41-1.34 (m, 2H), 1.17-1.07 (m, 2H); ¹³C NMR (100 MHz, CDCl₃) δ 156.3, 79.2, 55.1, 38.0, 34.5, 31.7 (2C), 28.8 (2C), 28.6 (3C), 26.3 (2C); IR (neat) vₘₐₓ: 2919, 2853, 1692, 1479, 1457, 1393, 1364, 1276, 1245, 1161, 1132, 881, 769; HRMS (ESI+): exact mass calculated for [M+Na]⁺ (C₁₄H₂₇NO₂Na) requires mlz 264.1934, found *m*/*z* 264.1934.

### Example 11: tert-Butyl (((1S,2S,4R)-bicyclo[2.2.1]heptan-2-yl)methyl)(methyl)carbamate (3j)

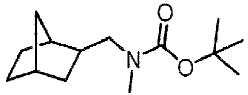

Prepared with 4 equiv. *N,N,N',N'*-tetramethyldiaminomethane according to general procedure A followed by the protection of the amino group by a Boc protective group; 79% yield; ¹H NMR (400 MHz, CDCl₃) δ 3.23-3.09 (m, 1H), 2.97-2.72 (m, 4H),2.21 (s, 1H), 2.02 (s, 1H), 1.75-1.68 (m, 1H), 1.53-1.46 (m, 2H), 1.45 (s, 9H), 1.33-1.28 (m, 2H), 1.16-1.06 (m, 3H), 1.00 (br s, 1H); ¹³C NMR (100 MHz, CDCl₃) δ 156.2, 79.2, 53.3, 40.8, 38.6, 36.6, 35.2, 34.9, 34.1, 29.9, 29.2, 28.6 (3C); IR (neat) vₘₐₓ: 2951, 2870, 1696, 1454, 1396, 1365, 1158, 1128; HRMS (ESI+): exact mass calculated for [M+Na]⁺ (C₁₄H₂₅NO₂Na) requires *m*/*z* 262.1778, found *m*/*z* 262.1779.

### Example 12: N-Benzyl-1-((1S,4R)-bicyclo[2.2.1]heptan-2-yl)methanamine (3k)

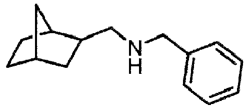

Prepared according to general procedure A, except for the fact that 1.5 equiv. *N,N,N',N'-*tetrabenzyldiaminomethane were used; 39% yield; ¹H NMR (400 MHz, CDCl₃) δ 7.35-7.29 (m, 4H), 7.27-7.21 (m, 1H), 3.78 (s, 2H), 2.50 (dd, *J* = 11.5, 8.1 Hz, 1H), 2.34 (dd, *J* = 11.5, 6.9 Hz, 1H), 2.19 (br s, 1H), 2.07 (br s, 1H), 1.67-1.58 (m, 1H), 1.54-1.46 (m, 2H), 1.46-1.30 (m, 2H), 1.29-1.24 (m, 1H), 1.21-0.99 (m, 4H); ¹³C NMR (100 MHz, CDCl₃) δ 140.9, 128.5 (2C), 128.2 (2C), 126.9, 55.3, 54.3, 42.6, 39.6, 36.4, 36.4, 35.5, 30.1, 29.0; IR (neat) vₘₐₓ: 2947, 2868, 2801, 1453, 1122, 733, 698; HRMS (ESI+): exact mass calculated for [M+H]⁺ (C₁₅H₂₂N) requires *m*/*z* 216.1747, found *m*/*z* 216.1745.

### Example 13: N-Methyl-5-phenylpentan-1-amine (3l)

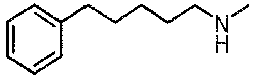

Prepared with 4 equiv. *N,N,N',N'*-tetramethyldiaminomethane according to general procedure A; 91% yield; ¹H NMR (400 MHz, CDCl₃) δ 7.30-7.24 (m, 2H), 7.21-7.15 (m, 3H), 2.62 (t, *J* = 7.8 Hz, 2H), 2.56 (t, *J* = 7.2 Hz, 2H), 2.42 (s, 3H), 1.69-1.60 (m, 2H), 1.56-1.47 (m, 2H), 1.42-1.33 (m, 2H), 0.91 (br s, 1H); ¹³C NMR (100 MHz, CDCl₃) δ 142.8, 128.5 (2C), 128.4 (2C), 125.7, 52.3, 36.7, 36.0, 31.5, 30.0, 27.1; IR (neat) vₘₐₓ: 3025, 2927, 2854, 2788, 1454, 1381, 1308, 811, 744, 698; HRMS (ESI+): exact mass calculated for [M+H]⁺ (C₁₂H₂₀N) requires *m*/*z* 178.1590, found *m*/*z* 178.1590.

### Example 14: N-Methyl-3-phenylpropan-1-amine (3m)

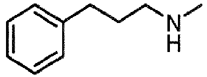

Prepared with 4 equiv. *N,N,N',N'*-tetramethyldiaminomethane according to general procedure A; 82% yield (50 mmol: 86% yield); ¹H NMR (400 MHz, CDCl₃) δ 7.30-7.25 (m, 2H), 7.22-7.15 (m, 3H), 2.66 (t, *J* = 7.8 Hz, 2H), 2.61 (t, *J* = 7.2 Hz, 2H), 2.43 (s, 3H), 1.86-1.77 (m, 2H), 1.02 (br s, 1H); ¹³C NMR (100 MHz, CDCl₃) δ 142.4, 128.5 (2C), 128.4 (2C), 125.9, 51.8, 36.7, 33.8, 31.7; IR (neat) vₘₐₓ: 3026, 2932, 2859, 1541, 1493, 1454, 1383, 1306, 1252, 748, 700; HRMS (ESI+): exact mass calculated for [M+H]⁺ (C₁₀H₁₆N) requires *m*/*z* 150.1277, found *m*/*z* 150.1274.

### Example 15: N-Methyl-3-(p-tolyl)propan-1-amine (3n)

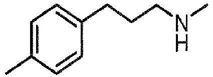

Prepared with 4 equiv. *N*,*N*,*N*',*N*'-tetramethyldiaminomethane according to general procedure A; 85% yield; ¹H NMR (400 MHz, CDCl₃) δ 7.09 (app s, 4H), 2.65-2.57 (m, 4H), 2.43 (s, 3H), 2.32 (s, 3H), 1.84-1.76 (m, 2H), 1.06 (br s, 1H); ¹³C NMR (100 MHz, CDCl₃) δ 139.3, 135.3, 129.1 (2C), 128.4 (2C), 51.8, 36.6, 33.3 31.8, 21.1; IR (neat) vₘₐₓ: 2025, 2855, 2794, 1515, 1457, 1381, 805; HRMS (ESI+): exact mass calculated for [M+H]⁺ (C₁₁H₁₈N) requires *m*/*z* 164.1434, found *m*/*z* 164.1435.

### Example 16: 3-(4-Chlorophenyl)-N-methylpropan-1-amine (3o)

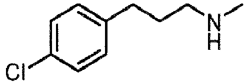

Prepared with 4 equiv. *N*,*N*,*N*',*N*'-tetramethyldiaminomethane according to general procedure A; 86% yield; ¹H NMR (400 MHz, CDCl₃) δ 7.26-7.21 (m, 2H), 7.13-7.09 (m, 2H), 2.65-2.56 (m, 4H), 2.42 (s, 3H), 1.82-1.73 (m, 2H), 0.99 (br s, 1H); ¹³C NMR (100 MHz, CDCl₃) δ 140.8, 131.6, 129.8 (2C), 128.5 (2C), 51.6, 36.7, 33.1, 31.6; IR (neat) vₘₐₓ: 2936, 2860, 1542, 1491, 1408, 1383, 1308, 1251, 1093, 1015, 833, 801; HRMS (ESI+): exact mass calculated for [M+H]⁺ (C₁₀H₁₅³⁵ClN) requires *m*/*z* 184.0888, found *m*/*z* 184.0880.

### Example 17: N-Methyl-3-(naphthalen-2-yl)propan-1-amine (3p)

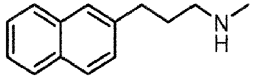

Prepared with 4 equiv. *N*,*N*,*N*',*N*'-tetramethyldiaminomethane according to general procedure A; 55% yield; ¹H NMR (400 MHz, CDCl₃) δ 7.83-7.74 (m, 3H), 7.63 (s, 1H), 7.48-7.38 (m, 2H), 7.36-7.32 (m, 1H), 2.87-2.80 (m, 2H), 2.69-2.62 (m, 2H), 2.44 (s, 3H), 1.97-1.86 (m, 2H), 1.10 (br s, 1H); ¹³C NMR (100 MHz, CDCl₃) δ 139.9, 133.8, 132.1, 128.0, 127.7, 127.5, 127.4, 126.5, 126.0, 125.2, 51.8, 36.7, 33.9, 31.6; IR (neat) vₘₐₓ: 2930, 2855, 1538, 1506, 1476, 1381, 1307, 816, 747; HRMS (ESI+): exact mass calculated for [M+H]⁺ (C₁₄H₁₈N) requires *mlz* 200.1434, found *m*/*z* 200.1438.

### Example 18: N-Benzyl-3-phenylpropan-1-amine (3q)

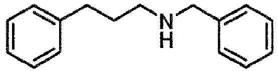

Prepared with 4 equiv. *N*,*N*,*N*',*N*'-tetrabenzyldiaminomethane according to general procedure A, 42% yield. ¹H NMR (400 MHz, CDCl₃) δ 7.27-7.26 (m, 3H), 7.22-7.20 (m, 4H), 7.14-7.11 (m, 3H), 3.73 (s, 2H), 2.62 (m, 2H), 1.86-1.73 (m, 2H,), 1.49 (br s, 1H); ¹³C NMR (150 MHz, CDCl₃) δ 142.3, 140.5, 128.5 (2C), 128.5 (2C), 128.5 (2C), 128.5 (2C), 128.3, 127.1, 125.9, 54.1, 49.0, 33.8, 31.8; IR (neat) vₘₐₓ: 3026, 2958, 2919, 2853, 1494, 1453, 1275, 1261, 749, 698; HRMS (ESI+): exact mass calculated for [M+H]⁺ (C₁₆H₂₀N) requires *m*/*z* = 226.1590, found *m*/*z* 226.1590.

### Example 19: N-(3-Phenylpropyl)butan-1-amine (3r)

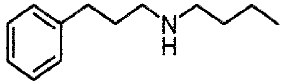

Prepared with 4 equiv. *N*,*N*,*N*',*N*'-tetrabutyldiaminomethane according to general procedure A; 86% yield; ¹H NMR (400 MHz, CDCl₃) δ 7.30-7.23 (m, 2H), 7.22-7.15 (m, 3H), 2.69-2.62 (m, 4H), 2.62-2.57 (m, 2H), 1.87-1.78 (m, 2H), 1.50-1.42 (m, 2H), 1.38-1.29 (m, 2H), 1.00-0.85 (m, 4H),; ¹³C NMR (100 MHz, CDCl₃) δ 142.4, 128.5 (2C), 128.4 (2C), 125.9, 49.9, 49.8, 33.9, 32.5, 32.0, 20.7, 14.2; IR (neat) vₘₐₓ: 2956, 2927, 2858, 1495, 1455, 1129, 699; HRMS (ESI+): exact mass calculated for [M+H]⁺ (C₁₃H₂₂N) requires *m*/*z* 192.1747, found *m*/*z* 192.1752.

### Example 20: N-(3-Phenylpropyl)prop-2-en-1-amine (3s)

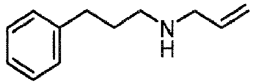

Prepared with 4 equiv. *N*,*N*,*N*',*N'-*tetraallyldiaminomethane according to general procedure A, 69% yield. ¹H NMR (400 MHz, CDCl₃) δ 7.30-7.26 (m, 2H), 7.20-7.16 (m, 3H), 5.91 (ddt, *J* = 16.3, 10.3, 6.0 Hz, 1H), 5.19-5.07 (m, 2H), 3.25 (m, 2H), 2.69-2.64 (m, 4H), 1.84 (m, 2H), 1.34 (br s, 1H); ¹³C NMR (100 MHz, CDCI3) δ 142.3, 137.0, 128.5 (2C), 128.5 (2C), 125.9, 116.0, 52.6, 49.1, 33.8, 31.9; IR (neat) vₘₐₓ: 2926, 2853, 1675, 1643, 1452, 1153, 1118, 917, 749, 700; HRMS (ESI+): exact mass calculated for [M+H]⁺ (C₁₂H₁₈N) requires *m*/*z* 176.1434, found *m*/*z* 176.1435.

### Example 21: N-Isobutyl-2-methyl-3-phenylpropan-1-amine (3t)

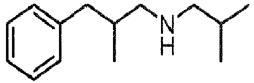

Prepared with 4 equiv. *N*,*N*,*N*',*N*'-tetraisobutyldiaminomethane according to general procedure A from *cis*-β-methylstyrene-61% yield, *trans-*β-methylstyrene-60% yield; ¹H NMR (400 MHz, CDCl₃) δ 7.30-7.24 (m, 2H), 7.21-7.14 (m, 3H), 2.73 (dd, *J* = 13.4, 6.0 Hz, 1H), 2.55 (dd, *J* = 11.7, 6.0 Hz, 1H), 2.47-2.35 (m, 4H), 2.00-1.88 (m, 1H), 1.78-1.66 (m, 1H), 1.08 (br s, 1H), 0.91-0.86 (m, 9H); ¹³C NMR (100 MHz, CDCl₃) δ 141.3, 129.3 (2C), 128.3 (2C), 125.9, 58.5, 56.4, 41.8, 35.4, 28.4, 20.8, 20.8, 18.1; IR (neat) vₘₐₓ: 3027, 2954, 2927, 1495, 1457, 1094, 739, 700; HRMS (ESI+): exact mass calculated for [M+H]⁺ (C₁₄H₂₄N) requires *m*/*z* 206.1903, found *m*/*z* 206.1904.

### Example 22: N,2-Dimethyl-3-phenylpropan-1-amine (3u)

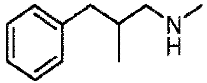

Prepared with 4 equiv. *N*,*N*,*N*',*N*'-tetramethyldiaminomethane according to general procedure A from *cis*-β-methylstyrene; 39% yield; ¹H NMR (400 MHz, CDCI3) δ 7.30-7.24 (m, 2H), 7.21-7.14 (m, 3H), 2.73 (dd, *J* = 13.4, 6.0 Hz, 1H), 2.53 (dd, *J* = 11.6, 6.0 Hz, 1H), 2.45-2.35 (m, 5H), 2.00-1.87 (m, 1H), 1.01 (br s, 1H), 0.89 (d, *J* = 6.7 Hz, 3H); ¹³C NMR (100 MHz, CDCl₃) δ 141.2, 129.3 (2C), 128.3 (2C), 125.9, 58.5, 41.7, 36.9, 35.4, 18.1; IR (neat) vₘₐₓ: 3174, 2960, 2924, 1668, 1630, 1545, 1454, 1384, 740, 701; HRMS (ESI+): exact mass calculated for [M+H]⁺ (C₁₁H₁₈N) requires *m*/*z* 164.1434, found *m*/*z* 164.1434.

### Example 23: N-Methyl-3,3-diphenylpropan-1-amine (3v)

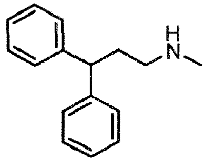

Prepared with 4 equiv. *N*,*N*,*N*',*N*'-tetramethyldiaminomethane according to general procedure A; 23% yield; ¹H NMR (600 MHz, CDCl₃) δ 7.30-7.24 (m, 8H), 7.17 (t, *J* = 6.9 Hz, 2H), 4.01 (t, *J* = 7.8 Hz, 1H), 2.54 (t, *J* = 7.1 Hz, 2H), 2.39 (s, 3H), 2.24 (app q, *J* = 7.4 Hz, 2H), 1.31 (br s, 1H); ¹³C NMR (150 MHz, CDCl₃) δ 144.9 (2C), 128.6 (4C), 127.9 (4C), 126.3 (2C), 50.6, 49.2, 36.6, 35.8; IR (neat) vₘₐₓ: 2815, 2766, 1493, 1445, 1367, 1024, 760, 699; HRMS

(ESI+): exact mass calculated for [M+H]⁺ (C₁₆H₂₀N) requires *m*/*z* 226.1590, found *m*/*z* 226.1592.

### Example 24: 12-(Methylamino)dodecyl acetate (3w)

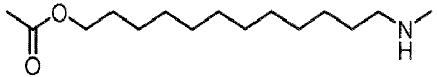

Prepared with 4 equiv. *N*,*N*,*N*',*N*'-tetramethyldiaminomethane according to general procedure A; 93% yield; ¹H NMR (600 MHz, CDCl₃) δ 4.04 (t, *J* = 6.8 Hz, 2H), 2.55 (t, *J* = 7.2 Hz, 2H), 2.42 (s, 3H), 2.04 (s, 3H), 1.64-1.57 (m, 2H), 1.50-1.43 (m, 2H), 1.36-1.20 (m, 17H); ¹³C NMR (150 MHz, CDCI3) δ 171.4, 64.8, 52.4, 36.7, 30.1, 29.7 (2C), 29.7, 29.7, 29.6, 29.4, 28.7, 27.5, 26.0, 21.2; IR (neat) vₘₐₓ: 2922, 2852, 1738, 1465, 1386, 1366, 1235, 1039; HRMS (ESI+): exact mass calculated for [M+H]⁺ (C₁₅H₃₂NO₂) requires *m*/*z* 258.2428, found *m*/*z* 258.2423.

### Example 25: Methyl 7-(methylamino)heptanoate (3x)

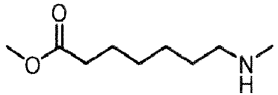

Prepared with 4 equiv. *N*,*N*,*N*',*N*'-tetramethyldiaminomethane according to general procedure A; 64% yield; ¹H NMR (400 MHz, CDCl₃) δ 3.65 (s, 3H), 2.54 (t, *J* = 7.1 Hz, 2H), 2.41 (s, 3H), 2.29 (t, *J* = 7.6 Hz, 2H), 1.66-1.57 (m, 2H), 1.51-1.42 (m, 2H), 1.37-1.29 (m, 4H), 1.06 (br s, 1H); ¹³C NMR (100 MHz, CDCl₃) δ 174.3, 52.2, 51.5, 36.7, 34.1, 29.9, 29.2, 27.1, 25.0; IR (neat) vₘₐₓ: 3359, 3199, 1664, 1557, 1396, 636; HRMS (ESI+): exact mass calculated for [M+H]⁺ (C₉H₂₀NO₂) requires *m*/*z* 174.1489, found *m*/*z* 174.1486.

### Example 26: Diethyl (9-(methylamino)nonyl)phosphonate (3y)

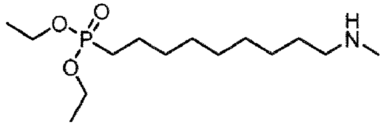

Prepared with 4 equiv. *N*,*N*,*N*',*N*'-tetramethylldiaminomethane according to general procedure A, 80% yield. ¹H NMR (600 MHz, CDCl₃) δ 4.11-4.03 (m, 4H), 2.54 (t, *J* = 7.2 Hz, 2H), 2.42 (s, 3H), 1.73-1.66 (m, 2H), 1.61-1.55 (m, 2H), 1.52 (br s, 1H), 1.47-1.45 (m, 2H), 1.36-1.34 (m, 2H), 1.30 (m, 14H); ¹³C NMR (150 MHz, CDCl₃) δ 61.5 (d, *J* = 6.5 Hz, 2C), 52.3, 36.6, 30.7 (d, *J* = 17.0), 30.0, 29.6, 29.4, 29.2, 27.4, 25.8 (d, *J* = 140.3), 22.5 (d, *J* = 5.2 Hz), 16.6 (d, *J* = 6.0 Hz, 2C); IR (neat) vₘₐₓ: 3427, 2925, 2853, 1465, 1386, 1235, 1054, 1024, 958; HRMS (ESI+): exact mass calculated for [M+H]⁺ (C₁₄H₃₃NO₃P) requires *m*/*z* 294.2193, found *m*/*z* 294.2192.

### Example 27: 12-(Methylamino)-N-(pyridin-4-yl)dodecanamide (3z)

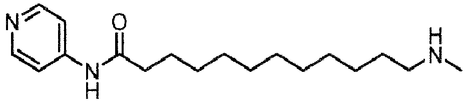

Prepared with 4 equiv. *N*,*N*,*N*',*N*'-tetramethytdiaminomethane according to general procedure A, 72% yield. ¹H NMR (400 MHz, CDCl₃) δ 8.48 (dd, *J* = 4.9, 1.4 Hz, 2H), 7.71 (br s, 1H), 7.49-7.48 (m, 2H), 3.48 (s, 1H), 2.57 (t, *J* = 7.2 Hz, 2H), 2.44 (s, 3H), 2.41-2.32 (m, 2H), 1.75-1.68 (m, 4H), 1.50-47 (m, 2H), 1.34-1.27 (m, 13H); ¹³C NMR (100 MHz, CDCl₃) δ 172.3, 150.8 (2C), 145.3, 113.6 (2C), 52.3, 37.9, 36.5, 29.9, 29.6, 29.5 (2C), 29.4, 29.3, 29.3, 27.4, 25.4; IR (neat) vₘₐₓ: 2925, 2853, 1703, 1594, 1522, 1329, 1296, 1210, 832; HRMS (ESI+): exact mass calculated for [M+H]⁺ (C₁₈H₃₂N₃O) requires *m*/*z* 306.2540, found *m*/*z* 306.2539.

### Example 28: 13-(Methylamino)tridecanenitrile (3aa)

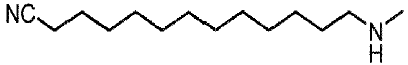

Prepared with 4 equiv. *N*,*N*,*N*',*N*'-tetramethyldiaminomethane according to general procedure A; 84% yield; ¹H NMR (600 MHz, CDCl₃) δ 2.55 (t, *J* = 7.1 Hz, 2H), 2.42 (s, 3H), 2.32 (t, *J =* 7.1 Hz, 2H), 1.64 (app quin, *J* = 7.5 Hz, 2H), 1.49-1.40 (m, 4H), 1.33-1.24 (m, 14H), 1.19 (br s, 1H); ¹³C NMR (150 MHz, CDCl₃) δ 120.0, 52.4, 36.7, 30.1, 29.7, 29.7, 29.6, 29.6, 29.4, 28.9, 28.8, 27.5, 25.5, 17.3; IR (neat) vₘₐₓ: 2922, 2852, 2243, 1465, 1426, 1382, 1308, 722; HRMS (ESI+): exact mass calculated for [M+H]⁺ (C₁₄H₂₉N₂) requires *m*/*z* 225.2325, found *m*/*z* 225.2324.

### Example 29: N-Benzyl-9-bromononan-1-amine (3ab)

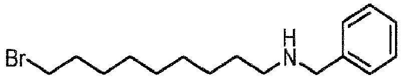

Prepared according to general procedure A, except that 1.5 equiv. *N,N,N',N'-*tetrabenzyldiaminomethane were used, 40% yield ¹H NMR (400 MHz, CDCl₃) δ 7.33 (d, *J* = 4.1 Hz, 4H), 7.25 (m, 1H), 3.80 (s, 2H), 3.40 (t, *J* = 6.9 Hz, 2H), 2.63 (t, *J* = 7.3 Hz, 2H), 1.88-1.81 (m, 2H,), 1.54-1.51 (m, 2H), 1.43-1.40 (m, 2H), 1.29-1.26 (m, 8H); ¹³C NMR (150 MHz, CDCl₃) δ 138.7, 128.7 (2C), 128.7 (2C), 127.5, 53.6, 49.9, 34.2, 32.9, 29.5, 28.8, 28.3, 27.3; IR (neat) vₘₐₓ: 2920, 2851, 2795, 1457, 1435, 734, 696; HRMS (ESI+): exact mass calculated for [M+H]⁺ (C₁₆H₂₇NBr) requires *m*/*z* 312.1321, found *m*/*z* 312.1319.

### Example 30: 12-(Methylamino)dodecan-1-ol (3ac)

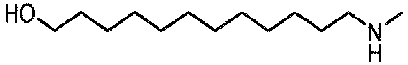

Prepared with 4 equiv. *N*,*N*,*N*',*N*'-tetramethyldiaminomethane according to general procedure A; 82% yield; ¹H NMR (400 MHz, CDCl₃) δ 3.63 (t, *J* = 6.6 Hz, 2H), 2.55 (t, *J* = 7.2 Hz, 2H), 2.42 (s, 3H), 1.60-1.51 (m, 2H), 1.51-1.42 (m, 2H), 1.38-1.24 (m, 18H); ¹³C NMR (100 MHz, CDCl₃) δ 63.2, 52.4, 36.7, 33.0, 30.1, 29.7 (3C), 29.7 (2C), 29.5, 27.5, 25.9; IR (neat) vₘₐₓ: 3304, 2918, 2850, 1680, 1468, 1387, 1202, 1180, 1134, 1059; HRMS (ESI+): exact mass calculated for [M+H]⁺ (C₁₃H₃₀NO) requires *m*/*z* 216.2322, found *m*/*z* 216.2314.

### Example 31: N¹-Butyl-N¹²-methyldodecane-1,12-diamine (3ad)

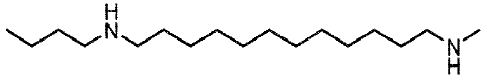

Prepared with 4 equiv. *N*,*N*,*N*',*N*'-tetramethyldiaminomethane according to general procedure A; 60% yield; ¹H NMR (400 MHz, CDCl₃) δ 2.61-2.52 (m, 6H), 2.42 (s, 3H), 1.51-1.41 (m, 6H), 1.37-1.24 (m, 18H), 1.00-0.85 (m, 5H); ¹³C NMR (100 MHz, CDCl₃) δ 52.4, 50.4, 50.0, 36.8, 32.5, 30.4, 30.1, 29.7 (7C), 27.6, 27.5, 20.7, 14.2; IR (neat) vₘₐₓ: 2921, 2850, 2811, 1466, 1377, 1128, 733; HRMS (ESI+): exact mass calculated for [M+H]⁺ (C₁₇H₃₉N₂) requires *m*/*z* 271.3108, found *m*/*z* 271.3108.

### Example 32: N¹,N¹-Diethyl-N¹²-methyldodecane-1,12-diamine (3ae)

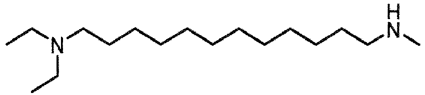

Prepared with 4 equiv. *N*,*N*,*N*',*N*'-tetramethyldiaminomethane according to general procedure A; 75% yield. ¹H NMR (400 MHz, CDCl₃) δ 2.55 (m, 6H), 2.43 (s, 3H), 2.44-2.40 (m, 2H), 1.68 (br s, 1H), 1.47-1.44 (m, 4H), 1.26 (s, 16H), 1.03 (t, *J* = 7.2 Hz, 6H); ¹³C NMR (150 MHz, CDCl₃) δ 53.0, 52.3, 46.9 (2C), 36.6, 30.0, 29.8, 29.7 (2C), 29.7 (2C), 27.8 (2C), 27.5, 26.9, 11.7 (2C); IR (neat) vₘₐₓ: 2967, 2922, 2851, 1466, 1381, 1201, 1130, 721; HRMS (ESI+): exact mass calculated for [M+H]⁺ (C₁₇H₃₉N₂O) requires *m*/*z* 271.3108, found *m*/*z* 271.3110.

### Example 33: (E)-N-Benzylundec-2-en-1-amine (6a)

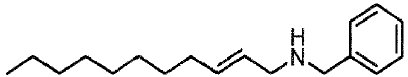

Procedure A, but with 1.5 equiv. *N*,*N*,*N*',*N*'-tetrabenzyldiaminomethane, 53% yield. ¹H NMR (600 MHz, CDCl₃) δ 7.32 (d, *J* = 4.5 Hz, 3H), 7.26-7.24 (m, 2H), 5.62-5.57 (m, 1H), 5.53 (dt, *J* = 15.3, 6.0 Hz, 1H), 3.78 (s, 2H), 3.21 (d, *J* = 6.0 Hz, 2H), 2.02 (app q, *J* = 6.9 Hz, 2H), 1.53 (br s, 1H), 1.37-33 (m, 2H,), 1.30-1.26 (m, 10H), 0.87 (t, *J =* 7.0 Hz, 3H); ¹³C NMR (150 MHz, CDCl₃) 5 140.5 (C), 133.3, 128.5 (2C), 128.4 (2C), 128.1, 127.0, 53.4, 51.3, 32.6, 32.0, 29.6, 29.5, 29.4, 29.3, 22.8, 14.3; IR (neat) vₘₐₓ: 3027, 2955, 2852, 1454, 969, 731, 698; HRMS (ESI+): exact mass calculated for [M+H]⁺ (C₁₈H₃₀N) requires *m*/*z* 260.2373, found *m*/*z* 260.2369.

### Example 34: (E)-3-Cyclohexyl-N-methylprop-2-en-1-amine (6b)

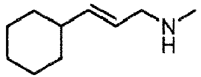

Procedure A with 4 equiv. *N*,*N*,*N*',*N*'-tetramethyldiaminomethane; 65% yield. ¹H NMR (400 MHz, CDCl₃) δ 5.54 (dd, *J* = 15.5, 6.3 Hz, 1H), 5.49-5.42 (m, 1H), 3.14 (d, *J* = 5.9 Hz, 2H), 2.41 (s, 3H), 1.94 (ddd, *J* = 14.1, 11.2, 3.0 Hz, 1H), 1.72 (dd, *J* = 9.2, 6.7 Hz, 4H), 1.66-1.62 (m, 1H), 1.33-1.01 (m, 6H); ¹³C NMR (100 MHz, CDCl₃) δ 138.9, 125.5, 54.0, 40.6, 35.9, 33.2 (2C), 26.4 (2C), 26.2; IR (neat) vₘₐₓ: 2921, 2849, 2789, 1447, 1379, 1315, 1286, 1254, 1031, 970; HRMS (ESI+): exact mass calculated for [M+H]⁺ (C₁₀H₁₉N) requires *m*/*z* 154.1590, found *m*/*z* 154.1590.

### Example 35: (E)-N-Benzyl-3-(cyclohex-1-en-1-yl)prop-2-en-1-amine (6c)

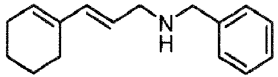

Procedure C with 1.5 equiv. *N*,*N*,*N*',*N*'-tetrabenzyldiaminomethane, 68% yield. ¹H NMR (400 MHz, CDCl₃) δ 7.35-7.30 (m, 4H), 7.28-7.23 (m, 1H), 6.17 (d, *J* = 15.7 Hz, 1H), 5.71 (br s, 1H), 5.69-5.59 (m, 1H), 3.80 (s, 2H), 3.32 (d, *J* = 6.3 Hz, 2H), 2.13-2.12 (m, 4H), 2.03 (s, 1H), 1.66 (ddd, *J* = 6.4, 4.6, 2.9 Hz, 2H), 1.63-1.54 (m, 2H); ¹³C NMR (100 MHz, CDCl₃) δ 140.2, 135.7, 135.5, 129.2, 128.6 (2C), 128.4 (2C), 127.1, 123.8, 53.3, 51.4, 26.0, 24.7, 22.7, 22.6; IR (neat) vₘₐₓ: 3025, 2922, 2855, 1493, 1450, 1072, 964, 731, 696; HRMS (ESI+): exact mass calculated for [M+H]⁺ (C₁₆H₂₂N) requires *m*/*z* 228.1747, found *m*/*z* 228.1744.

### Example 36: (E)-N-Benzyl-3-cyclopropylprop-2-en-1-amine (6d)

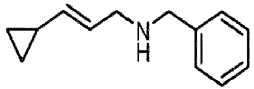

Procedure C with 1.5 equiv. *N*,*N*,*N*',*N*'-tetrabenzyldiaminomethane. 85%, 10:1 mixture of isomers. ¹H NMR (600 MHz, CDCl₃) δ 7.34-7.30 (m, 4H), 5.64 (dt, 1H, *J* = 15.0, 6.4 Hz), 5.14 (dd, *J* = 15.3, 8.7 Hz, 1H), 3.78 (s, 2H), 3.21 (d, *J* = 6.4 Hz, 2H), 1.40-1.38 (m, 1H), 0.70-0.67 (m, 2H), 0.36-0.34 (m, 2H); ¹³C NMR (150 MHz, CDCl₃) δ 140.5, 136.6, 128.5 (2C), 128.3 (2C), 127.0, 126.0, 53.4, 51.2, 13.6, 6.7 (2C); IR (neat) vₘₐₓ: 3083, 3064, 3025, 3005, 2958, 2924, 2853, 2807, 1495, 1453, 963, 735, 698; HRMS (ESI+): exact mass calculated for [M+H]⁺ (C₁₃H₁₈N) requires *m*/*z* 188.1434, found *m*/*z* 188.1433.

### Example 37: (E)-N-Methyl-2-propylhex-2-en-1-amine (6e)

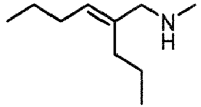

Procedure A with 4 equiv. *N*,*N*,*N*',*N*'-tetramethyldiaminomethane;, 41% yield. ¹H NMR (600 MHz, CDCl₃) δ 5.32-5.29 (m, 1H), 3.10 (s, 2H), 2.38 (s, 3H), 2.04-1.99 (m, 4H), 1.67 (s, 1H), 1.38 (dt, *J* = 23.9, 7.5 Hz, 4H), 0.90 (t, *J* = 7.3 Hz, 6H); ¹³C NMR (150 MHz, CDCl₃) δ 137.3, 127.1, 57.6, 35.9, 31.1, 29.8, 23.2, 21.9, 14.3, 14.1; IR (neat) vₘₐₓ: 3446, 2965, 2918, 1455, 1396, 764, 703; ; HRMS (ESI+): exact mass calculated for [M+H]⁺ (C₁₀H₂₁N) requires *m*/*z* 156.1747, found *m*/*z* 156.1743.

### Example 38: (E)-N-Methyl-3-(p-tolyl)prop-2-en-1-amine (6f)

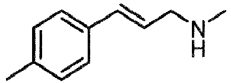

Procedure A with 4 equiv. *N*,*N*,*N*',*N*'-tetramethyldiaminomethane; 51% yield, 10:1 mixture of isomers. ¹H NMR (400 MHz, CDCl₃) δ 7.27 (d, *J* = 7.9 Hz, 2H), 7.11 (d, *J* = 7.9 Hz, 2H), 6.50 (d, *J* = 15.9 Hz, 1H), 6.24 (dt, *J* = 15.9, 6.3 Hz, 1H), 3.37 (dd, *J* = 6.3, 1.4 Hz, 2H), 2.48 (s, 3H), 2.33 (s, 3H), 1.40 (br s, 1H); ¹³C NMR (100 MHz, CDCl₃) δ 137.3, 134.5, 131.4, 129.4 (2C), 127.4, 126.3 (2C), 54.0, 36.2, 21.3; IR (neat) vₘₐₓ: 3045, 2922, 2853, 1669, 1513, 1451, 1382, 970, 766, 749; HRMS (ESI+): exact mass calculated for [M+H]⁺ (C₁₁H₁₆N) requires *m*/*z* 162.1277, found *m*/*z* 162.1274.

### Example 39: (E)-3-(4-Fluorophenyl)-N-methylprop-2-en-1-amine (6g)

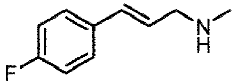

Procedure A with 4 equiv. *N*,*N*,*N*',*N*'-tetramethyldiaminomethane; 31% yield. ¹H NMR (700 MHz, CDCI3) δ 7.34-7.32 (m, 2H), 6.99 (t, *J* = 8.7 Hz, 2H), 6.49 (d, *J* = 15.9 Hz, 1H), 6.20 (dt, *J* = 15.9, 6.3 Hz, 1H), 3.36 (dd, *J* = 6.3, 0.9 Hz, 2H), 2.48 (s, 3H), 1.25 (br s, 1H); ¹³C NMR (175 MHz, CDCl₃) δ 162.3 (d, *J* = 246 Hz), 133.5 (d, *J* = 3.3 Hz), 130.2, 128.3 (d, *J* = 2.1 Hz), 127.8 (d, *J* = 7.9 Hz, 2C), 115.6 (*J* = 21.5 Hz, 2C), 53.9, 36.2; IR (neat) vₘₐₓ: 2360, 2177, 2045, 1509, 1227, 763, 749; HRMS (ESI+): exact mass calculated for [M+H]⁺ (C₁₀H₁₃N) requires *m*/*z* 166.1027, found *m*/*z* 166.1021.

### Example 40: (E)-2-(4-(3-(Methylamino)prop-1-en-1-yl)phenyl)acetonitrile (6h)

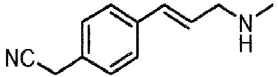

Procedure B with 4 equiv. *N*,*N*,*N*',*N*'-tetramethyldiaminomethane; 37% yield. ¹H NMR (600 MHz, CDCl₃) δ 7.38 (d, *J* = 8.2 Hz, 2H), 7.27-7.26 (m, 2H), 6.52 (d, *J* = 15.9 Hz, 1H), 6.31 (dt, *J* = 15.9, 6.2 Hz, 1H), 3.73 (s, 2H), 3.39 (dd, *J* = 6.3, 1.4 Hz, 2H), 2.48 (s, 3H), 1.63 (s, 1H); ¹³C NMR (150 MHz, CDCl₃) δ 137.0, 130.3, 129.2, 128.6, 128.1 (2C), 126.9 (2C), 117.8, 53.7, 36.0, 23.3; IR (neat) vₘₐₓ: 2981, 2898, 1670, 1512, 1361, 1275, 1261, 972, 764, 750; HRMS (ESI+): exact mass calculated for [M+H]⁺ (C₁₂H₁₅N) requires *m*/*z* 187.1230, found *m*/*z* 187.1224.

### Example 41: (E)-N,2-Dimethyl-3-phenylprop-2-en-1-amine (6i)

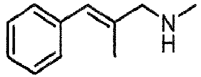

Procedure A with 4 equiv. *N*,*N*,*N*',*N*'-tetramethyldiaminomethane; 48% yield. ¹H NMR (600 MHz, CDCl₃) δ 7.33 (t, *J* = 7.6 Hz, 2H), 7.27 (d, *J* = 7.3 Hz, 2H), 7.20 (t, *J* = 7.3 Hz, 1H), 6.44 (s, 1H), 3.30 (s, 2H), 2.47 (s, 3H), 1.90 (d, *J* = 0.9 Hz, 3H), 1.57 (br s, 1H); ¹³C NMR (150 MHz, CDCl₃) δ 138.1, 136.8, 129.0 (2C), 128.2 (2C), 126.3, 126.0, 60.4, 35.9, 16.7; IR (neat) vₘₐₓ: 3024, 2925, 2853, 2794, 2770, 2714, 1680, 1491, 1448, 1387, 1357, 1037, 748, 699; HRMS (ESI+): exact mass calculated for [M+H]⁺ (C₁₁H₁₆N) requires *m*/*z* 162.1277, found *m*/*z* 162.1277.

### Example 42: Methyl (E)-7-(benzylamino)hept-5-enoate (6j)

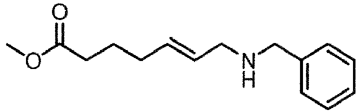

Procedure A, but with with 1.5 equiv. *N*,*N*,*N*',*N*'-tetrabenzyidiaminomethane; 41% yield, 12:1 mixture of isomers. ¹H NMR (400 MHz, CDCl₃) δ 7.26 (d, *J* = 4.5 Hz, 4H), 7.19-7.17 (m, 1H), 5.53-5.51 (m, 2H), 3.73 (s, 2H), 3.61 (s, 3H), 3.17 (dd, *J* = 3.7, 1.0 Hz, 2H), 2.26 (t, *J* = 7.5 Hz, 2H), 2.03-2.01 (m, 2H), 1.82 (br s, 1H), 1.67 (p, *J* = 7.5 Hz, 2H); ¹³C NMR (100 MHz, CDCl₃) δ 174.2, 140.3, 131.7, 129.4, 128.5 (2C), 128.4 (2C), 127.1, 53.4, 51.6, 51.1, 33.5, 31.8, 24.6; IR (neat) vₘₐₓ: 2926, 2797, 1734, 1602, 1451, 1436, 1154, 1121, 970, 734, 698; HRMS (ESI+): exact mass calculated for [M+H]⁺ (C₁₅H₂₂NO₂) requires *m*/*z* 248.1645, found *m*/*z* 248.1644.

### Example 43: (E)-8-(Methylamino)oct-6-enenitrile (6k)

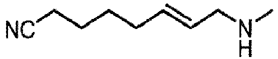

Procedure B with 4 equiv. *N*,*N*,*N*',*N*'-tetramethyldiaminomethane; 97% yield, 10:1 mixture of isomers. ¹H NMR (400 MHz, CDCl₃) δ 5.54 (td, *J* = 5.1, 3.0 Hz, 2H), 3.16-3.14 (m, 2H), 2.41 (s, 3H), 2.33 (t, *J* = 7.0 Hz, 2H), 2.07 (dd, *J* = 12.2, 7.2 Hz, 2H), 1.70-1.63 (m, 2H), 1.57-1.50 (m, 2H), 1.32 (br s, 1H); ¹³C NMR (100 MHz, CDCl₃) δ 131.4, 129.4, 119.8, 53.7, 36.0, 31.5, 28.3, 24.9, 17.2; IR (neat) vₘₐₓ: 2932, 2857, 2790, 1541, 1460, 1381, 1262, 1032, 974, 764, 750; HRMS (ESI+): exact mass calculated for [M+H]⁺ (C₉H₁₇N₂) requires *m*/*z* 153.1386, found *m*/*z* 153.1386.

### Example 44: (E)-11-(Methylamino)undec-9-en-1-ol (6l)

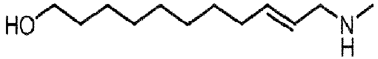

Procedure B with 4 equiv. *N*,*N*,*N*',*N*'-tetramethyldiaminomethane, stirred for 5 h at 75 °C instead of 16 h; 59% yield. ¹H NMR (600 MHz, CDCl₃) δ 5.53 (dtd, 2H), 3.62 (t, *J* = 6.6 Hz, 2H), 3.14 (d, *J* = 6.1 Hz, 2H), 2.40 (s, 3H), 2.01 (q, *J* = 6.8 Hz, 2H), 1.63-1.53 (m, 4H), 1.35-1.24 (m, 10H); ¹³C NMR (150 MHz, CDCl₃) δ 133.2, 128.0, 63.1, 53.8, 35.9, 32.9, 32.4, 29.4, 29.3, 29.1, 25.8; IR (neat) vₘₐₓ: 3405, 3368, 2925, 2854, 1541, 1460, 1382, 1260, 1059, 971; HRMS (ESI+): exact mass calculated for [M+H]^{*} (C₁₂H₂₆NO) requires *m*/*z* 200.2009, found *m*/*z* 200.2009.

### Example 45: (E)-N-Methyl-4-(triisopropylsilyl)but-2-en-1-amine (6m)

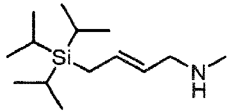

Procedure B with 4 equiv. *N*,*N*,*N*',*N*'-tetramethyldiaminomethane; stirred at room temperature instead of 75 °C, 34% yield. ¹H NMR (400 MHz, CDCl₃) δ 5.68-5.60 (m, 1H), 5.46-5.38 (m, 1H), 3.12 (dd, *J* = 6.5, 0.8 Hz, 2H), 2.40 (s, 3H), 1.59 (dd, *J* = 8.0, 1.1 Hz, 2H), 1.15 (br s, 1H), 1.05 (s, 9H); ¹³C NMR (100 MHz, CDCl₃) δ 130.1, 126.9, 54.4, 36.0, 18.8 (6C), 15.5, 11.2 (3C); IR (neat) vₘₐₓ: 2940, 2889, 2864, 1461, 1381, 1253, 1154, 966, 809, 749, 701, 658; HRMS (ESI+): exact mass calculated for [M+H]⁺ (C₁₄H₃₂N) requires *m*/*z* 242.2294, found *m*/*z* 242.2295.

### C-C Bond Formation on the Reaction Intermediate

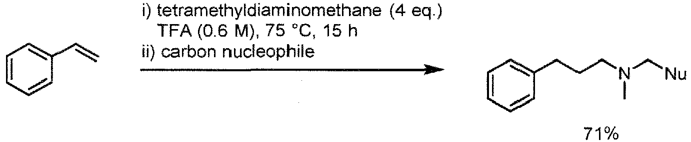

### Example 46: 4-(Methyl(3-phenylpropyl)amino)butan-2-one (8a)

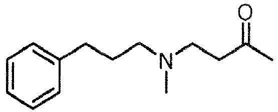

Following procedure A, styrene was subjected to hydroaminomethylation-conditions using *N*,*N*,*N*',*N*'-tetramethyldiaminomethane. After the hydroaminomethylation reaction and cooling to 23 °C, acetone (55 equiv.) was added directly to the reaction mixture and the resulting solution was stirred at 23 °C for 8 h. After this time, volatile components were removed under reduced pressure and the residue was dissolved in chloroform. The resulting solution was treated with a 1 M aqueous solution of sodium hydroxide until basic and the aqueous phase was subsequently extracted with chloroform (3 ×). The combined organic phases were dried over anhydrous sodium sulfate, the dried solution was filtered and the filtrate was concentrated under reduced pressure. The crude residue was purified by flash column chromatography over silica gel (dichloromethane/MeOH/NH₄OH 18/1/0.15) to afford the title compound.
71% yield; ¹H NMR (400 MHz, CDCl₃) δ 7.30-7.24 (m, 2H), 7.20-7.14 (m, 3H), 2.68-2.54 (m, 6H), 2.39-2.33 (m, 2H), 2.20 (s, 3H), 2.15 (s, 3H), 1.83-1.74 (m, 2H); ¹³C NMR (100 MHz, CDCl₃) δ 208.2, 142.4, 128.5 (2C), 128.4 (2C), 125.9, 57.2, 52.2, 42.1, 41.8, 33.7, 30.3, 29.1; IR (neat) vₘₐₓ: 2942, 2847, 2793, 1711, 1495, 1454, 1356, 1161, 747, 700; HRMS (ESI+): exact mass calculated for [M+H]⁺ (C₁₄H₂₂NO) requires *m*/*z* 220.1696, found *m*/*z* 220.1696.

### Example 47: 3-(Methyl(3-phenylpropyl)amino)-1-phenylpropan-1-one (8b)

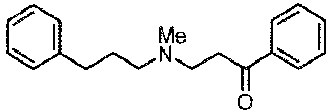

Following procedure A, styrene was subjected to hydroaminomethylation-conditions using 4 equivalents of *N*,*N*,*N*',*N*'-tetramethyldiaminomethane. After the hydroaminomethylation reaction and cooling to 23 °C, acetophenone (10 equiv.) was added directly to the reaction mixture and the resulting solution was stirred at 23 °C for 8 h. After this time, volatile components were removed under reduced pressure and the residue was dissolved in chloroform. The resulting solution was treated with a 1 M aqueous solution of sodium hydroxide until basic and the aqueous phase was subsequently extracted with chloroform (3 x). The combined organic phases were dried over anhydrous sodium sulfate, the dried solution was filtered and the filtrate was concentrated under reduced pressure. The crude residue was purified by flash column chromatography over silica gel (dichloromethane/MeOH/NH₄OH 18/1/0.15) to afford the title compound.
59% yield; ¹H NMR (600 MHz, CDCl₃) δ 7.96-7.95 (m, 2H), 7.58-7.55 (m, 1H), 7.48-7.45 (m, 2H), 7.28-7.26 (m, 2H), 7.19-7.16 (m, 3H), 3.15-3.12 (m, 2H), 2.85-2.83 (m, 2H), 2.63-2.61 (m, 2H), 2.44-2.42 (m, 2H), 2.29 (s, 1H), 1.81 (dt, *J* = 15.0, 7.6 Hz, 2H); ¹³C NMR (150 MHz, CDCl₃) δ 199.6, 142.3, 137.1, 133.2, 128.7 (2C), 128.6 (2C), 128.4 (2C), 128.2 (2C), 125.9, 57.2, 52.7, 42.4, 36.7, 33.7, 29.1; IR (neat) vₘₐₓ: 2939, 2849, 2794, 1682, 1598, 1495, 1450, 1206, 1180, 745, 696; HRMS (ESI+): exact mass calculated for [M+H]⁺ (C₁₉H₂₄NO₃) requires *m*/*z* 282.1852, found *m*/*z* 282.1854.

### Example 48: N-Methyl-3-phenyl-N-(2,4,6-trimethoxybenzyl)propan-1-amine (8c)

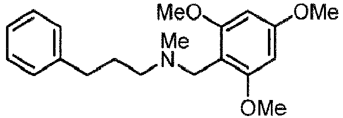

Following procedure A, styrene was subjected to hydroaminomethylation-conditions using 4 equivalents of *N*,*N*,*N*',*N*'-tetramethyidiaminomethane. After the hydroaminomethylation reaction and cooling to 23 °C, 1,3,5-trimethoxybenzene (15 equiv.) was added directly to the reaction mixture and the resulting solution was warmed up to 50 °C and stirred for 8 h. After this time, volatile components were removed under reduced pressure and the residue was dissolved in chloroform. The resulting solution was treated with a 1 M aqueous solution of sodium hydroxide until basic and the aqueous phase was subsequently extracted with chloroform (3 x). The combined organic phases were dried over anhydrous sodium sulfate, the dried solution was filtered and the filtrate was concentrated under reduced pressure. The crude residue was purified by flash column chromatography over silica gel (dichloromethane/MeOH/NH₄OH 18/1/0.15) to afford the title compound.
54% yield; ¹H NMR (600 MHz, CDCl₃) δ 7.29-7.26 (m, 2H), 7.20 (t, *J* = 7.4 Hz, 1H), 7.16 (d, *J* = 7.0 Hz, 2H), 6.11 (s, 2H), 4.24 (m, 1H), 4.12 (m, 1H), 3.82 (s, 3H), 3.79 (s, 6H), 3.09 (br s, 1H), 2.80 (br s, 1H), 2.68 (t, *J* = 6.2 Hz, 2H), 2.61 (s, 3H), 2.28 (br s, 1H), 2.17 (br s, 1H); ¹³C NMR (150 MHz, CDCl₃) δ 163.3 (2C), 161.1, 160.6, 140.1, 128.7 (2C), 128.5 (2C), 126.5, 90.6 (2C), 55.9 (2C), 55.6, 55.1, 47.5, 39.6, 33.2, 25.6; IR (neat) vₘₐₓ: 3397, 2941, 2845, 2613, 1609, 1594, 1458, 1421, 1232, 1204, 1152, 1055, 1033, 951, 819; HRMS (ESI+): exact mass calculated for [M+H]⁺ (C₂₀)H₂₈NO₃) requires *m*/*z* 330.2064, found *m*/*z* 330.2055.

### General Procedure for Comparative Examples 1 to 4

A round bottom flask charged with N,N-tetramethyldiaminomethane (4 equiv. with respect to the alkene) and a magnetic stir-bar was cooled to 0 C. After this, sulfuric or phosphoric acid (1.8 equivalents with respect to the alkene) and acetic acid were added, followed by the alkene in one portion, in a 0.6M concentration with respect to the acetic acid. The flask was sealed, placed in an oil bath and heated to 75 °C for the time indicatded in tables 1 and 2. Subsequently, the volatile components were removed at reduced pressure. The crude mixture was then treated with aqueous sodium hydroxide (1-2 mol/L) and chloroform and stirred vigorously for one hour. After that, aqueous sodium hydroxide was added unil the mixture reached pH 12. The resulted biphasic mixture was then separated and the aqueous phase extracted with chloroform (3x200 mL). The combined organic phases were dried over sodium sulfate and concentrated under reduced pressure.

**Table 1**

| 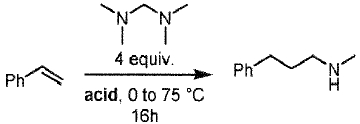 | | | | |
|---|---|---|---|---|
| | Temperature | Time [h] | Acid | NMR yield |
| Example 14 | 75 °C | 16h | TFA | 86% isolated |
| Comparative Example 1 | 75 °C | 16h | H₂SO₄ (1.8 equiv) in AcOH | <40% |
| Comparative Example 2 | 75 °C | 16h | H₃PO₄ (1.8 equiv) in AcOH | <40% |

**Table 2**

| 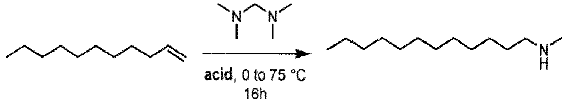 | | | | |
|---|---|---|---|---|
| | Temperature | Time [h] | Acid | NMR yield |
| Example 8 | 75 °C | 16h | TFA | 61% isolated |
| Comparative Example 3 | 75 °C | 5h | H₂SO₄ (1.8 equiv) in AcOH | 10% |
| Comparative Example 4 | 75 °C | 5h | H₃PO₄ (1.8 equiv) in AcOH | 10% |

As can be taken from Table 1 and Table 2, the hydroaminomethylation reaction according to the present invention resulted in high yields and high selectivity (Examples 8 and 14). In contrast, the hydroaminomethylation using sulfuric acid or phosphoric acid in acetic acid resulted in low yields and the formation of side products (Comparative Examples 1 to 4).

**Table 3**

| 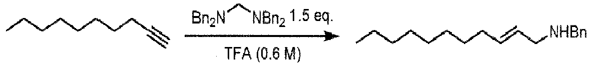 | | | | | | |
|---|---|---|---|---|---|---|
| | Solvent system | Equiv. TFA | Concentration of alkyne | Temperature | Time (h) | NMR Yield |
| Example 33 | TFA | 22.4 | 0.6M | 75 | 15 | 53 (isolated) |
| Example 49* | TFA/DCE 1:1 | 22.4 | 0.3M | 75 | 15 | 75 |
| Example 50* | TFA/MeCN 1:1 | 22.4 | 0.3M | 75 | 15 | 53 |
| Example 51* | TFA/Toluene 1:1 | 22.4 | 0.3M | 75 | 15 | 64 |
| Example 52** | TFA/DCE 1:1 | 11.2 | 0.6M | 75 | 15 | 70 |
| Example 53* | TFA/DCE 1:1 | 15 | 0.45M | 75 | 15 | 53 |

| | | | | | | |
|---|---|---|---|---|---|---|
| * The Examples were carried out according to General Procedure C, but with the concentration of the TFA, the additional solvent and the alkyne as indicated. ** The Example was carried out according to General Procedure C. | | | | | | |

As can be seen in Table 3, high yields of the amine were obtained, i.e. above 50%, in a hydroaminomethylation reaction carried out in TFA alone and also for solvent mixtures of TFA and an organic solvent, such as dichloroethane (DCE), acetonitrile (MeCN) and toluene.

## Claims

1. A process for producing an amine via a hydroaminomethylation reaction of a non-aromatic C-C double bond or C-C triple bond, said process comprising
a step of reacting a compound comprising a non-aromatic C-C double bond or C-C triple bond with a reactive component which is obtainable by combining an aminal with an acidic medium comprising trifluoroacetic acid,
wherein the aminal contains two amino groups independently selected from a secondary and a tertiary amino group that are linked by a methylene group, and at least one of the amino groups carries a hydrogen atom at a carbon atom bound in α-position to its nitrogen atom.

2. The process according to claim 1, wherein the acidic medium is neat TFA.

3. The process according to claim 1, wherein the acidic medium comprises a further organic solvent, more preferably selected from the group consisting of benzene which may be substituted, dichloroethene, acetonitrile, ester solvents, dimethylformamide, dimethylsulfoxide, chloroform, dichloromethane, and alcohols.

4. The process according to any of claims 1 to 3, wherein the acidic medium comprises TFA in an amount of 1 molar equivalent or more, for each mole of non-aromatic C-C double bond or C-C triple bond that is subjected to the hydroaminomethylation reaction.

5. The process according to any of claims 1 to 4, wherein the aminal is represented by the following formula (I): 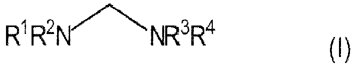 wherein
R¹ and R³ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof;
R² and R⁴ are independently selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof;
and R¹ and R² or R³ and R⁴, respectively, may be bonded to each other to form a ring together with the nitrogen atom to which they are bound, or one of R¹ and R² and one of R³ and R⁴ may be bonded to each other to form a ring together with the methylene group and the nitrogen atoms to which they are bound;
and wherein at least one of the amino groups -NR¹R² and -NR³R⁴ carries a hydrogen atom at a carbon atom in α-position to the nitrogen atom.

6. The process according to claim 5, wherein R¹, R², R³ and R⁴ are independently selected from the group consisting of methyl, ethyl, propyl, isopropyl, butyl, isobutyl, benzyl and allyl, or wherein R¹ and R² or R³ and R⁴, respectively, are bonded to each other to form a ring together with the nitrogen atom to which they are bound, more preferably a piperidin-1-yl ring.

7. The process according to claim 5 or 6, wherein R¹, R², R³ and R⁴ are selected such that the groups -NR¹R² and -NR³R⁴ are identical.

8. The process according to any of claims 5 to 7, wherein R¹, R², R³ and R⁴ are identical.

9. The process according to any of claims 1 to 8, wherein the aminal is combined in an amount of 1 molar equivalent or more, for each mole of non-aromatic C-C double bond or C-C triple bond that is subjected to the hydroaminomethylation reaction, with the acidic medium.

10. The process according to any of claims 1 to 9, wherein the compound comprising a non-aromatic C-C double bond is represented by the following formula (II): 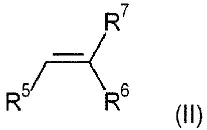 wherein R⁵, R⁶ and R⁷ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, and wherein R⁵ and R⁶ may be bonded to each other to form a ring together with the carbon atoms to which they are bound.

11. The process according to any of claims 1 to 9, wherein the compound comprising a C-C triple bond is represented by the following formula (III): 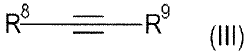
wherein R⁸ and R⁹ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, and
wherein R⁸ and R⁹ may be bonded to each other to form a ring together with the carbon atoms to which they are bound.

12. The process according to any of claims 1 to 11, wherein said step of reacting the compound comprising a non-aromatic C-C double bond or C-C triple bond with the reactive component is carried out at a reaction temperature in the range of 0°C to 150°C.

13. The process according to any of claims 1 to 12, wherein the step of reacting the compound comprising a non-aromatic C-C double bond or a C-C triple bond with the reactive component is carried out in the absence of a metal catalyst.

14. The process according to any of claims 1 to 13, which further comprises a step wherein water is added to the reaction mixture resulting from the step of reacting a compound comprising a non-aromatic C-C double bond or C-C triple bond with the reactive component.

15. The process according to any of claims 1 to 13, which further comprises a step wherein an organic nucleophilic reactant is added to the reaction mixture resulting from the step of reacting a compound comprising a non-aromatic C-C double bond or a C-C triple bond with the reactive component.
